(19) Europäisches Patentamt
European Patent Office
Office européen des brevets

(11) EP 1 144 412 B1

(12) # EUROPEAN PATENT SPECIFICATION

(45) Date of publication and mention of the grant of the patent:
**29.09.2004 Bulletin 2004/40**

(21) Application number: **99964663.1**

(22) Date of filing: **28.12.1999**

(51) Int Cl.7: **C07D 475/04**, C07D 475/08, A61K 31/519, A61P 35/00

(86) International application number:
**PCT/EP1999/010320**

(87) International publication number:
**WO 2000/039129 (06.07.2000 Gazette 2000/27)**

(54) **IMMUNOSUPPRESSIVE EFFECTS OF PTERIDINE DERIVATIVES**

IMMUNSUPPRESSIVE WIRKUNGEN VON PTERIDINDERIVATEN

EFFETS IMMUNOSUPPRESSEURS DE DERIVES DE LA PTERIDINE

(84) Designated Contracting States:
**AT BE CH CY DE DK ES FI FR GB GR IE IT LI LU MC NL PT SE**

(30) Priority: **28.12.1998 US 113989 P**

(43) Date of publication of application:
**17.10.2001 Bulletin 2001/42**

(73) Proprietor: **4 AZA Bioscience nv**
**3000 Leuven (BE)**

(72) Inventors:
- **WAER, Mark, Joseph, Albert**
  **B-3001 Heverlee (BE)**
- **HERDEWIJN, Piet, André, Maurits, Maria**
  **B-3111 Rotselaar/Wezemaal (BE)**
- **PFLEIDERER, Wolfgang, Eugen**
  **D-78464 Konstanz (DE)**

(74) Representative: **Bird, Ariane**
**Bird Goen & Co,**
**Klein Dalenstraat 42A**
**3020 Winksele (BE)**

(56) References cited:
**EP-A- 0 108 890**
**EP-A- 0 290 819**
**EP-A- 0 362 645**
**EP-A- 0 956 855**
**WO-A-95/31987**
**WO-A-95/32203**
**WO-A-98/04558**
**WO-A-98/08516**
**US-A- 5 992 713**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention relates to a pharmaceutical composition for use in the treatment of autoimmuno disorders or for the treatment and/or prevention of transplant-rejections and/or the treatment of inflammatory diseases comprising as active ingredient one or more pteridine derivatives as defined in claim 1.

The invention further relates to combined pharmaceutical preparations comprising one or more pteridine derivates and one or more known immunosuppressant, and to a group of novel compounds (pteridine derivates) as defined in claim 13.

Those compounds disclaimed from claim 13 relate to disclosures according to reference 8 and 9 (see page 13)

Several pteridine derivates are known in nature and used in the preparation of medicines, for example as described in EP-A-108 890. Other medical uses of derivates of pteridine are described in WO 95-31987 as NO-synthase inhibitors for example for the treatment of diseases caused by a high nitrogen monoxide level. Further, WO-95-32203 describes also the use of tetrahydropteridine derivates as NO-synthase inhibitors.

Both above-mentioned WO publications disclose also the use of specific pteridine derivates in the treatment of patholigically low blood pressure, in particular septic shock and combined with cytokines in tumor therapy and in transplant-rejection diseases.

Although some of these pteridine derivates are claimed as potentially active for the treatment of transplant-rejection diseases, direct evidence for their effectiveness is lacking. Overall there still is a need for specific and highly active immunosuppressive compounds, in particular immunosupressive compounds active in the cosignal pathway.

A first object of the invention is to provide a pharmaceutical composition having high immunosuppressive activity. Another object of the invention is to provide a combined immunosuppressive preparation which causes a superadditive effect, comprising a pteridine derivate of the invention and other known immunosuppressants.

Another further object of the invention is to provide immunosuppressive compounds, which are active in a minor dose, in order to decrease the considerable treatment costs.

Known immunosuppressive compounds are for example cyclosporine A, subsituted xanthines, tacrolimus (FK 506), rapamycine (RPM), leflunomide, mofetil, adrenocortical steroids, cytotoxic drugs and antibody preparations.

The immunosuppressive effect of cyclosporine A (CyA) is already known since 1972. However, due to its nephrotoxicity and several other side effects CyA has not been able to establish itself as the optimal and final drug of choice.

Methylxanthines, for example pentoxifylline (PTX), are known having immunosuppressive effects in vitro.

Recently (Lin Y. et al, Transplantation 63 (1997) it has been found that the co-medication of an immunosuppressive compound such as cyclosporine A (CyA) or FK506 or RPM (rapamycine) with a methylxanthine derivative, in particular A802715 (7-propyl-1(5-hydroxy-5-methylhexyl)-3-methylxanthine) leads to a superadditive increase in the immunosuppressive action.

Likewise, other substituted, in particular substituted 8-phenylxanthines have been found to possess immunosuppressive effects in vitro (application EP 98.201323.7).

The present invention relates in particular to the application of a group pteridine derivates and their pharmaceutical salts, possessing unexpectedly desirable pharmaceutical properties, i.c. are higly active immunosuppressive agents, are usefull in the treatment in transplant rejection and/or in the treatment of inflammatory diseases.

The invention demonstrates the immunosuppressive effects of pharmaceutical compositions for the treatment of autoimmuno disorders or of transplant-rejections comprising one or more pteridine derivatives of the above formula (I) or salts thereof.

The term pharmaceutically acceptable addition salt as used hereinbefore defines the non-toxic, therapeutically active addition salt forms which the compounds of formula (I) may form. The compounds of formula (I) having basic properties may be converted into the corresponding therapeutically active, non-toxic acid addition salt forms by treating the free base form with a suitable amount of an appropiate acid following conventional procedures. Examples of appropiate acids aaare for example, inorganic acids, for example, hydrohalic acid, e.g. hydrochloric, hydrobromic and the like acids, sulfuric acid, nitric acid, phosphoric acid and the like; or organic acids, such as, for example, acetic, propanic, hydroxyacetic, 2-hydroxypropanic, 2-oxopropanic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

The compounds of formula (I) having acidic properties may be converted in a similar manner into the corresponding therapeutically active, non-toxic base addition salt forms. Examples of such base addition salt forms are, for example, the sodium, potassium, calcium salts, and also the salts with pharmaceutically acceptable amines such as, for example, ammonia, alkylamines, benzathine, N-methyl-D-glucamine, hydrabamine, amino acids, e.g. arginine, lysine. The term pharmaceutically acceptable addition salts also comprises the solvates which the compounds of formula (I) may form, e.g. the hydrates, alcoholates and the like.

The term stereochemically isomeric forms as used hereinbefore defines the possible different isomeric as well as conformational forms which the compounds of formula (I) may possess. Unless otherwise mentioned or indicated, the chemical designation of compounds denotes the mixture of all possible sterochemically and conformationally isomeric forms, said mixtures containing all diastereomers, enantiomers and/or conformers of the basic molecular structure. All stereochemically isomeric forms of the compounds of formula (I) both in pure form or in admixture with each other are intended to be embraced within the scope of the present invention.

Some compounds of the present invention may exist in different tautomeric forms and all such tautomeric forms are intended to be included within the scope of the present invention.

The compounds of the present invention show a broad spectrum profile as is evidenced by the results obtained in the diversity of test procedures cited hereinbefore.

An advantageous feature of the compounds of the present invention resides in their excellent oral activity; the present compounds when administered orally have been found to be practically equipotent with the same being administered subcutaneously.

A particularly important asset of most of the present compounds is their lack of sedating properties at therapeutic dose levels, a troublesome side effect associated with many antihistaminic and antiallergic compounds. The non-sedating properties of the present compounds can be demonstrated, for example, by the results obtained in studying the sleep - wakefulness cycle of the rat (Psychopharmacology, 97, 436-442, (1989)).

Another interesting feature of the present compounds relates to their fast onset of action and the favorable duration of their action.

In view of their useful properties the subject compounds may be formulated inot various pharmaceutical forms for administration purposes. To prepare the antiallergic compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in adminstration, tablets and capsules represent the most advantageous oral dosage unit form, in which case solid pharmaceutical carriers are obviously employed. For parental compositions, the carrier will usually comprise steriel water, at least in large part, through other ingredients, for example to aid solubility, may be included. Injectable solutions, for example, may be prepared in which the carrier comprises saline solution, glucose solution or a mixture of saline and glucose solution. Injectable suspensions may also be prepared in which case appropiate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may' facilitate the administration to the skin and/or may be helpful for preparing the desired compositions. These compositions may be administered in various ways, e.g. as a transdermal patch, as a spot-on or as an ointment. Acid addition salts of the subject compounds due to their increased ater solubility over the corresponding base form, are obviously more suitable in the preparation of aqueous compositions.

It is especially advantageous to formulate the aforementioned pharmaceutical compositions in dosage unit form for ease of administration and uniformaty of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The present invention also relates to a method of treating warm-blooded animals suffering from said allergic diseases by administering to said warm-blooded animals an effective antiallergic amount of a compound of formula (I).

In general it is contemplated that an effective antiallergic amount would be from about 0.001 mg/kg to about 20 mg/kg body weight, and more preferably from about 0.01 mg/kg to about 5 mg/kg body weight.

The following examples are intended to illustrate and not to limit the scope of the present inventionl in all its aspects.

**Experimental Part**

2-amino-4-n-pentyloxy-6-styrylpteridine (**1**).

A mixture of 2-amino-6-chloro-4-n-pentyloxypteridine [1] (1.5 g, 5.6 mmoles) palladium acetate (63 mg, 0.28 mmoles), tri-o-tolylphosphane (682 mg, 2.24 mmoles), cuprous iodide (53 mg, 0.28 mmoles), styrene (1,3 ml., 11.3 mmoles) and triethylamine 3.1 ml, 22 mmoles) was stirred in dry acetonitrile (50 ml.) under reflux for 90 hours. It was evaporated and the residue purified by silica gel column chromatography with $CHCl_3$. The product fraction was evaporated to give 1.37 g (72%) of an orange powder. Recrystallization from EtOAc/hexane. M.p. 127-128°C.

2-Amino-6-(1,2-dibromophenethyl)-4-n-pentyloxypteridine (**2**).

To a solution of **1** (1.0 g, 2.94 mmoles) in chloroform (50 ml.) was added a 2 M bromine solution in chloroform (2.2 ml., 4.4 mmoles) and then the mixture stirred at room temperature for 7 hours. It was diluted with chloroform (50 ml.), washed with a saturated aqueous $Na_2SO_3$ solution (100 ml.) and dried over $Na_2SO_4$. It was evaporated, the residue treated with little toluene, filtered, washed with ether and dried in a vacuum desiccator to give 0.84 g (57%) yellow powder.

**2-Amino-4,7-dimethoxy-6-styrylpteridine (3).**

A suspension of **2** (0.3 g, 0.6 mmoles) is abs. Methanol (10 ml.) was treated with 1 M methanolic sodium methoxide (3 ml., 3 mmoles) and then refluxed for 4 hours. It was diluted with chloroform (100 ml.), washed with saturated aqueous $NH_4Cl$ solution and water and then the solution dried over $Na_2SO_4$. The filtrate was evaporated and the residue purified by silica gel column chromatography in chloroform. The product fraction was evaporated to give 50 mg. (26%) of a yellow powder, M.p. 197-198°C.

$O^4$-Methyl-biopterin (4).

To a solution of $N^2$, 1',2'-O-triacetyl-biopterin (1,0 g; 2.75 mmoles), triphenylphosphane (12,08 g, 4.13 mmoles) and methanol (0.15 ml., 3.7 mmoles) in dry dioxane (30 ml.) was added diisopropyl azodicarboxylate (0.81 g, 4.11 mmoles) and after stirring for 1.5 hours at room temperature evaporated to dryness. The residue was purified by silica gel column chromatography eluting with EtOAc/$CHCl_3$ (1:4). The product fraction was evaporated and dried in vacuum to give 0.4 g (38%) of $N^2$, 1',2'-O-triacetyl-$O^4$-methylbiopterin.
Deacetylation of the reaction product (0.28 g, 0.74 mmoles) was done by stirring in abs. Methanol (20 ml.) and triethylamine (4 ml.) for 24 hours. Evaporation to dryness, treatment of the residue with ether, filtration and drying gave 0.172 g (83%) of **4**. M.p. 160-161°C (Decomp.).

2-amino-4-hydroxylamino-6-phenylpteridines (13).

A suspension of 2,5,6-triamino-4-methoxypyrimidine dihydrochloride (1 g, 4 mmoles) in methanol (40 ml.) was heated to boiling and then a solution of phenylglyoxalmonoxime (1 g, 6.6 mmoles) in methanol (10 ml.) added dropwise. A clear solution is obtained from which on reflux for 2 hours a precipitate separated out. The solid was filtered off (hydrochloride salt), suspended in water (30 ml) and then neutralized to pH 8 by conc. ammonia. The precipitate was collected, washed with water and ethanol and dried at 100°C to give a yellow powder. Yield: 0.84 g (82%).

General procedures for the synthesis of 2,6-diamino-4-dialkylamino-5-p-chlorophenylazopyrimidines.

A solution of 2,6-diamino-4-dialkylamino-5-p-chlorophenylazopyrimidine [7] (5.0 g, 16.6 mmoles) in DMF (50ml) and the appropriate amine (dimethylamine in ethanol (50%), diethylamine, di-n-propylamine, dibenzylamine, morpholine, piperidine, pyrrolidine, piperazine, N-methylpiperazine) (10.0 g) was heated in an oilbath to 70°C for 5 hours. Then water (50 ml) was added, cooled and the yellow precipitate collected, washed with water and dried. Recrystallization from EtOH or DMF/water. Yield: 55-90%.

General procedure for the synthesis of 2,5,6-triamino-4-dialkylaminopyrimidines.

A suspension of 2,6-diamino-4-dialkylamino-5-p-chlorophenylazopyrimidine (3.28 g, 10 mmoles) in methanol (70 ml) and conc. ammonia (10 ml) was reduced in a shaking apparatus under $H_2$ atmosphere in presence of Raney nickel catalyst (3.5 g) for 2 days. The catalyst was filtered off under argon atmosphere and then the filtrate evaporated

in vacuo to dryness. The residue was treated with ether to remove the p-chloroaniline, filtered and then the solid stirred in methanolic HCl (10%, 50 ml) overnight. The dihydrochloride salt was collected and dried in a vacuum desiccator over KOH. Yield: 85-90%.

General procedure for the synthesis of 2-amino-4-dialkylamino-6-arylpteridines (14-16, 21-49)

To a boiling solution of the 2,5,6-triamino-4-dialkylaminopyrimidine dihydrochloride salt (5 mmoles) in MeOH (20 ml) was added a solution of the arylglyoxalmonoxime (7.5 mmoles) in MeOH (10 ml) dropwise and then the mixture heated under reflux for 3 hours. After cooling the suspension or solution was made alkaline by conc. ammonia to pH 9 and the resulting precipitate filtered off, washed with water and dried. Recrystallization was done from EtOH and DMF/$H_2O$, respectively, to give a yellow solid. Yield: 50-85%.

Further compounds 50-66 were prepared according to the above described syntheses and tested.

References

[1] D. Mohr, Z. Kazimierczuk, W. Pfeiderer, *Helv. Chim. Acta* **1992**, *75*, 2317.
[2] L. Classen, O. Manasse, *Ber. Dtsch. Chem. Ges.,* **1887**, *20*, 2194.
[3] I. Lalezari, *J. Org. Chem.*, **1968**, *33*, 4281.
[4] J.W.G. DeMeester, H.C. van der Plas, *J. Heterocycl. Chem.*, **1987,** *24*, 441.
[5] W. Borsche, *Ber. Dtsch. Chem. Ges.*, **1929,** *62*, 1360.
[6] J.R. Piper, J.A. Montgomery, J. *Org. Chem.,* **1977**, *42*, 208.
[7] W.R. Boon, T. Leigh, Brit., 342, Aug. 13, 1952; *C.A. ,* **1953**, *47*
[8] Boon, W.R. J.Chem. Soc. 1957, 2146-2158
[9] Kaldrikyan: Arm. Khim. Zh. 1976, 29, 337-341.


Materials and methods

Various models may be used for testing an immunosuppressive effect. In vivo, for example, different transplantation models are available. They are strongly influenced by different immunogenicities, depending on the donor and recipient species used and depending on the nature of the transplanted organ. The survival time of transplanted organs can thus be used to measure the suppression of the immune response. In vitro, there exist also various models. The most used are lymphocyte activation tests. Usually activation is measured via lymphocyte proliferation. Inhibition of proliferation thus always means immunosuppression under the experimental conditions applied. There exist different stimuli for lymphocyte activation:

- coculture of lymphocytes of different species (MLR = mixed lymphocyte reaction): lymphocytes expressing different minor and major antigens of the HLA-DR type (= alloantigens) activate each other non-specifically.
- CD3 assay: here there is an activation of the T-lymphocytes via an exogenously added antibody (OKT3). This antibody reacts against the CD3 molecule located on the lymphocyte membrane. This molecule has a costimulatory function. The interaction anti-CD3 (= OKT3)-CD3 results in T-cell activation which proceeds via the $Ca^2$+/calmodulin/calcineurin system and can be inhibited by CyA.
- CD28 assay: here specific activation of the T-lymphocyte goes also via an exogenously added antibody against the CD28 molecule. This molecule is also located on the lymphocyte membrane, and delivers strong costimulatory signals. This activation is $Ca^2$-independent and thus cannot be inhibited by CyA.

Reagents

All derivatives were dissolved in 0.5 ml DMSO and further diluted in culture medium before use in in vitro experiments. The culture medium consisted of RPMI-1640 + 10% FCS.

Mixed Lymphocyte Reaction

Peripheral blood mononuclear cells (PBMC) were isolated from heparinized peripheral blood by density gradient centrifugation over Lymphoprep (Nycomed, Maorstua, Norway). Allogeneic PBMC or EBV-transformed human B cells [RPMI1788 (ATCC name CCL156)] which strongly express B7-1 and B7-2 were used as stimulator cells after irradiation with 30 Gy. MLR was performed in triplicate wells. After 5 days incubation at 37EC, 1 µCi [$^3$H]-thymidine

was added to each cup. After a further 16 hours incubation, cells were harvested and counted in a β-counter.

The percent suppression of proliferation by drugs was counted using the formula

$$\text{Percent inhibition} = \frac{(\text{cpm + drugs}) - \text{cpm Cult. Med}}{(\text{cpm - drugs}) - \text{cpm Cult. Med.}} \times 100$$

T cell purification

T cells were purified by removing non-T cells. Briefly,monocytes were removed by cold agglutination. The resulting lymphoid cells were further purified by a cell enrichment immunocolumn [Cellect Human T (Biotex, Edmonton, Alberta, Canada)] by a process of negative selection. More than 95% of the B cells were removed with this procedure. After depletion, the resulting T cell preparation was highly purified explaining these cells could not be activated by PHA or rIL-2 alone at concentrations capable of stimulating RBMC prior to deletion.

Measurements of T cell proliferations induced by anti-CD3 mAb + PMA or anti-CD28 mAb + PMA

Highly purified T cells ($10^6$/ml) were stimulated by immobilized anti-CD3 àr anti-CD28 mAb in the presence of PMA. Anti-CD3 mAb (CLB-CD3; CLB, Amsterdam, The Netherlands) were fixed on the 96-microwell plates by incubating the wells with 50 µl of mAb solution (1/800 dilution in culture medium). Anti-CD28 mAb (CLB-CD28; CLB, Amsterdam, The Netherlands) 50 µl (1/650 dilution in culture medium) was added directly to the wells. Further, 20 µl PMA (Sigma, St. Louis, MO, USA) solution (final concentration: 0.5 ng/ml) was added. Subsequently, 20 µl of immunosuppressants were added by serial dilution in triplicate wells. Finally 100 µl of the T cell suspension ($10^6$/ml) was added. After 48-hour incubation at 37EC in 5% $CO_2$ 20 Φl BrdU (100 ΦM solution) (Cell Proliferation Elisa, Boehringer-Mannheim Belgium) was added to each well. After a further overnight incubation the T cell proliferation was measured using a colorimetric immunoassay for qualification of cell proliferation based on measurements of the incorporation of BrdU during DNAsynthesis. The optical density (OD) was measured by a Behring EL311 plate reader at 450 nm (reference wavelength: 690 nm). The percent suppression of proliferation by drugs was counted using the formula:

$$\text{Per cent inhibition} = \frac{(\text{OD + drugs}) - (\text{OD Cult. Med.})}{(\text{OD - drugs}) - (\text{OD Cult. Med.})} \times 100$$

<u>In vitro immunosuppressive effect of Pteridine Derivatives as measured with the MLR and with tests involving polyclonal T cell proliferation induced by anti-CD3 mAb + PMA or anti-CD28 mAb + PMA</u> (table II)

- Table II shows the IC50 values of the various substances in the MLR. The IC50 value represents the lowest concentration of the substances that resulted in a 50% suppression of the MLR. These concentrations are divided into for subranges i.e.

  0 stands for concentrations of at least 151µM,

  + stands for concentrations 16-150 µM,

  ++ stands for concentrations 1-15 µM,

  +++ stands for concentrations lower than 1 µM,

- Column III shows the IC50 value of the various substances for the anti-CD3 mAb + PMA pathway and row IV the IC50 values of the various substances for the anti-CD28 mAb + PMA pathway.
- As a comparison the values of other immunosuppressants: CsA, FK506, Rapamycin, Leflunomide and Mycophenolic acid methatroxate (MTX) and 5-Fluoro-uracil (5-FU) in table III are given as well.

First, most of the pteridine classes (I) according to the invention contain compounds with a clear suppressive effect in the MLR (mixed lymphocyte reaction). The MLR is considered as an in vitro analogue of the transplant rejection as it is based on the recognition of allogeneic MHC (major histocompatibility antigens) on the stimulator leucotyes, by responding lymphocytes. Various established immunosuppressive drugs are known to suppress the MLR, and were also shown in this description.

From these data it can be deduced that the pteridine derivatives are effective in clinical situations where other immunosuppressants are active as well.

These include the prevention and/or treatment of organ transplant rejection, the prevention and/or treatment of both rejection and the occurrence of graft-versus-host-disease after BM transplantation; the prevention and/or treatment of autoimmune diseases including diabetes mellitus, multiple sclerosis, glomerulonephritis, rheumatoid arthritis, psoriasis systemic diseases such as vasculitis; scleroderma, polymyositis, autoimmune endocrine disorders (thyroiditis), ocular diseases (uveitis), inflammatory bowel diseases (Crohn's disease, colitis ulcerosa), autoimmune liver diseases (autoimmune hepatitis, primary biliary cirrhosis) autoimmune pneumonitis and auto-immune carditis.

Whereas cyclosporine A and FK506 are only active in the anti-CD3 + PMA test, the pteridine derivatives according to the invention were active, not only in the anti-CD3 + PMA but also in the anti-CD28 + PMA test. It has been shown that the latter is Ca-calmodulin resistant, and resistant to CsA and FK506. The anti-CD28 + PMA pathway has also been called the cosignal pathway and is important to induce energy and even tolerance in T cells. Moreover, representative compounds have been found to be active in an whole blood assay.

Under the term "organ" in the description is understood all organs or parts of organs (even several) in mammals, in particular humans, for example kidney, heart, skin, liver, muscle, cornea, bone, bone marrow, lung, pancreas, intestine or stomach.

After organ transplantation, rejection of the transplanted organ by the recipient occurs (host-versus-graft reaction). After bone marrow transplantation, also rejection of the host by the grafted cell may occur (graft-versus-host reaction). Rejection reactions mean all reactions of the recipient body or of the transplanted organ which in the end lead to cell or tissue death in the transplanted organ or adversely affect the functional ability and viability of the transplanted organ or adversely affect the functional ability and viability of the transplanted organ or the recipient. In particular, this means acute and chronic rejection reactions.

Auto-immune disorders include, inter alia, systemic lupus erythematosus, rheumatoid arthritis, psoriasis, pemphigus, atopic dermatitis, myositis, multiple sclerosis, nephrotic syndrome (in particular glomerulonephritis), ulcerative colitis or juvenile diabetes.

An additive or synergetic effect of pteridine derivatives and other immunosuppressants may be anticipated. This may be especially, although not exclusively the case for combinations with CyA or FK 506 as the latter are not suppressive in the aCD28 pathway of T cell activation (table III) whereas most Ptedridine derivatives are.

The invention further relates to the use of cyclosporin A or FK506 or Rapamycine and at least one pteridine derivative according to the invention for the production of a pharmaceutical for inhibiting the replication of viruses such as picorna-, toga-, bunya-, orthomyxo-, paramyxo-, rhabdo-, retro-, arena-, hepatitis B-, hepatitis C-, hepatitis D-, adeno-, vaccinia-, papilloma-, herpes-, varicella-zoster-virus or human immunodeficiency virus (HIV); or for treating of cancer such as lung cancers, leukaemia, ovarian cancers, sarcoma, Kaposi's sarcoma, meningioma, colon cancers, lymp node tumors, glioblastoma multiforme, prostate cancers or skin carcinoses.

The invention further relates to the use of cyclosporin A or FK506 or rapamycin and at least one pteridine derivative of the general formula (I) for the production of a pharmaceutical for the treatment of human after organ transplantation or of (auto)immune disorders.

Hence, the advantage to associate pteridine with other immunosuppressants may be that, first, the therapeutic spectrum of action of the individual components is quantitatively and qualitatively broadened. Secondly that it allows, by means of a dose reduction without reduced efficacy but with increased safety, that the treatment of immune disorders which were hitherto no indication for immunosuppressive therapy as a result of side effects may be considered. At the same time, the therapy costs can be decreased to an appreciable extent.

As a comparison, known pteridine derivatives are submitted to the same test conditions as the pteridine derivatives of the invention. These compounds and the results thereof are given in table IV and show no particular immunosuppressive activity.

As been stated above the invention also relates to new pteridine derivatives as such, in particular the compounds 1,2,3,6, 14-16 and 21-66 and their pharmaceutically acceptable salts.

| Compound n° | MLR | aCD3 | aCD28 |
|---|---|---|---|
| 1 2-amino-4-pentoxy-6-styrylpteridine 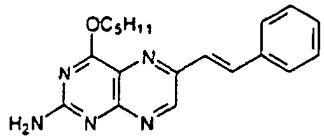 | 15 | 110 | 80 |
| 2 2-amino-4-n-pentoxy-6-(1,2-dibromo-2-phenylethyl)pteridine 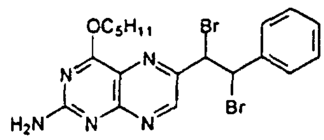 | 12 | 4 | 12 |
| 3 2-amino-4-methoxy-6-styryl-7-methoxypteridine 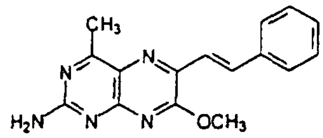 | 25 | 160 | 100 |
| 4 2-amino-4-methoxy-6-(1,2-dihydroxypropyl)pteridine 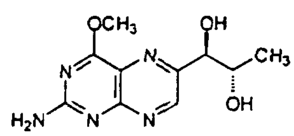 | >200 | 140 | 110 |
| 5 2,4-diamino-6-phenyl pteridine 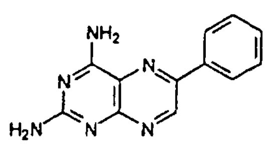 | >200 | >200 | >200 |
| 6 2,4-diamino-6-phenyl-7-methylpteridine 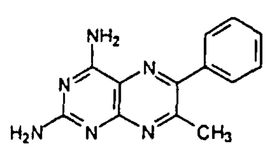 | >200 | >200 | >200 |

| | | | | |
|---|---|---|---|---|
| 7 | 2,4-diamino-6-(4-tolyl)pteridine<br>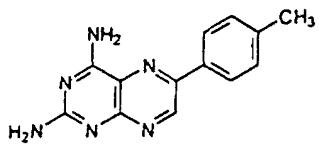 | >200 | 110 | 25 |
| 8 | 2,4-diamino-6-(4-methoxyphenyl)pteridine<br>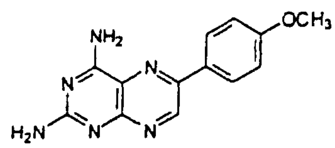 | >150 | 125 | 22 |
| 9 | 2,4-diamino-6-(4-chlorophenyl)pteridine<br>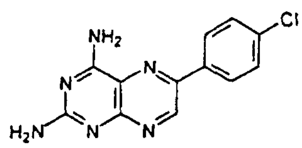 | 125 | 110 | 110 |
| 10 | 2-hydroxyethylamino-4-amino-6-phenylpteridine<br>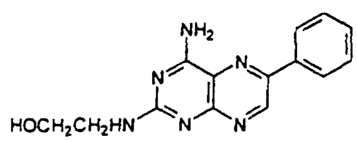 | 125 | 125 | 125 |
| 11 | 2-hydroxyethylamino-4-amino-6-(4-tolyl)pteridine<br>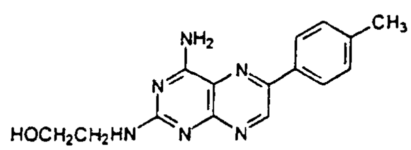 | >200 | 200 | 160 |
| 12 | 2-hydroxyethylamino-4-amino-6-(4-methoxyphenyl)pteridine<br>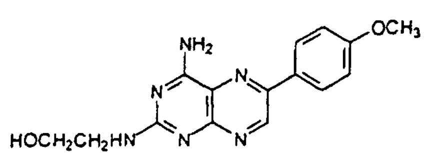 | >200 | >200 | 160 |

| | | | | |
|---|---|---|---|---|
| 13 | 2-amino-4-hydroxylamino-6-phenylpteridine<br>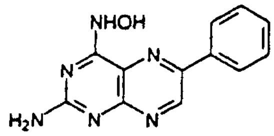 | 140 | >200 | >200 |
| 14 | 2-amino-4-dimethylamino-6-phenylpteridine<br>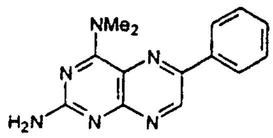 | 5.0 | 15 | 15 |
| 15 | 2-amino-4-dimethylamino-6-(4-tolyl)pteridine<br>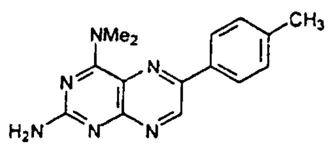 | 3.6 | 4.5 | 3.9 |
| 16 | 2-amino-4-dimethylamino-6-(4-methoxyphenyl)pteridine<br>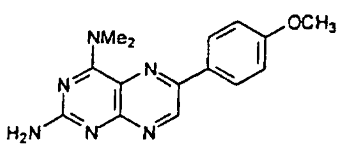 | 12 | 7.5 | 7.5 |
| 17 | 2,4-diamino-6-methoxyethoxymethyl pteridine<br>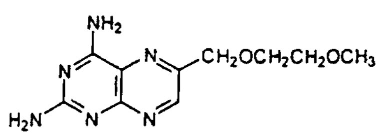 | >200 | 180 | 18 |
| 18 | 2,4-diamino-6-decyloxymethyl pteridine<br>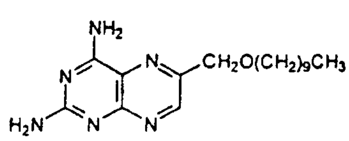 | >200 | 152 | 100 |
| 19 | 2,4-diamino-6-benzylaminomethyl pteridine | 50 | 123 | 118 |

| | | | | |
|---|---|---|---|---|
| | 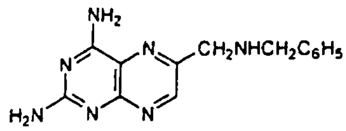 | | | |
| 20 | 2,4-diamino-6-dimethyl aminomethyl pteridine<br>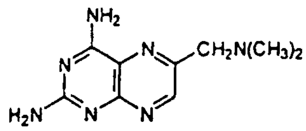 | >200 | 170 | 100 |
| 21 | 2-amino-4-diethylamino-6-phenylpteridine<br>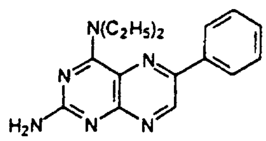 | 15 | 80 | 20 |
| 22 | 2-amino-4-diethylamino-6-(4-chlorophenyl) ptēridine<br>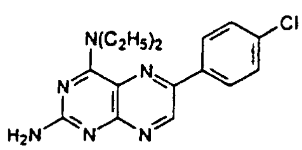 | 17 | 50 | 20 |
| 23 | 2-amino-4-diethylamino-6-(4-methoxyphenyl) pteridine<br>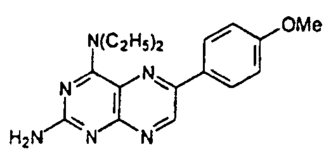 | 12 | 20 | 20 |
| 24 | 2-amino-4-diethylamino-6-(3,4-dimethoxyphenyl) pteridine<br>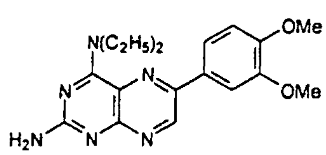 | 0.08 | 0.5 | 0.1 |
| 25 | 2-amino-4-dibenzylamino-6-phenyl pteridine<br>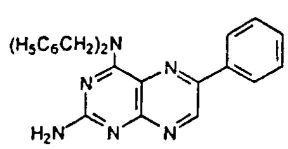 | 75 | 25 | 25 |

| | | | | |
|---|---|---|---|---|
| 26 | 2-amino-dibenzylamino-6-(4-chlorophenyl) pteridine<br>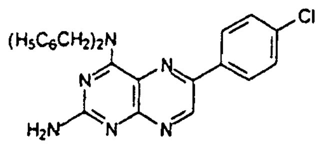 | 100 | 75 | 20 |
| 27 | 2-amino-4-dibenzylamino-6-(4-methoxyphenyl) pteridine<br>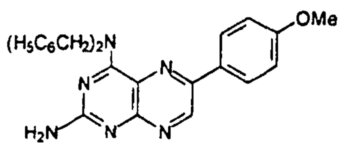 | 50 | 20 | 15 |
| 28 | 2-amino-4-dibenzylamino-6-(3,4-dimethoxyphenyl) pteridine<br>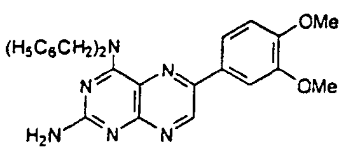 | 3.5 | 4.3 | 3.2 |
| 29 | 2-amino-4-dipropylamino-6-phenylpteridine<br>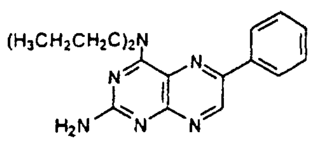 | 15 | | 25 |
| 30 | 2-amino-4-dipropylamino-6-(4-chlorophenyl)pteridine<br>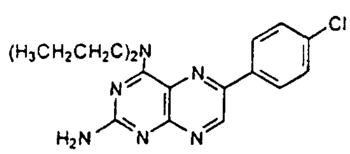 | 13 | 9.7 | 6.7 |
| 31 | 2-amino-4-dipropylamino-6-(4-methoxyphenyl)pteridine<br>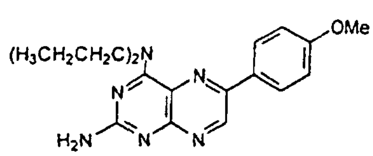 | 8.5 | 5.7 | 2.8 |

| | | | | |
|---|---|---|---|---|
| **32** | **2-amino-4-dipropylamino-6-(3,4-dimethoxyphenyl)pteridine**<br>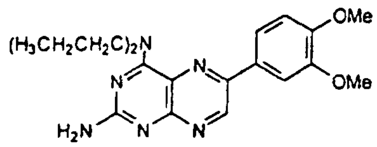 | 2.2 | 0.8 | 0.7 |
| **33** | **2-amino-4-morpholino-6-phenylpteridine**<br>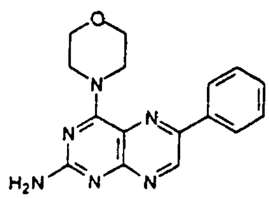 | 15 | 100 | 20 |
| **34** | **2-amino-4-morpholino-6-(4-chlorophenyl)pteridine**<br>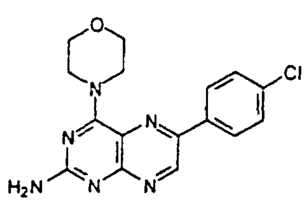 | 12 | 20 | 20 |
| **35** | **2-amino-4-morpholino-6-(4-methoxyphenyl)pteridine**<br>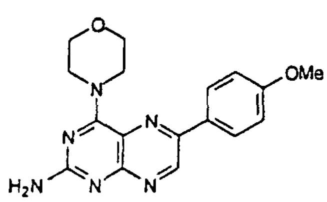 | 15 | 125 | 20 |
| **36** | **2-amino-4-morpholino-6-(3,4-dimethoxyphenyl)pteridine**<br>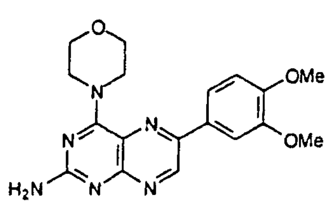 | 0.1 | 0.4 | 0.3 |

| | | | | |
|---|---|---|---|---|
| 37 | 2-amino-4-piperidino-6-phenylpteridine<br>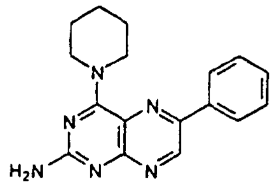 | 15 | 80 | 20 |
| 38 | 2-amino-4-piperidino-6-(4-chlorophenyl)pteridine<br>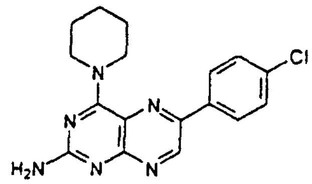 | 15 | 70 | 25 |
| 39 | 2-amino-4-piperidino-6-(4-methoxyphenyl)pteridine<br>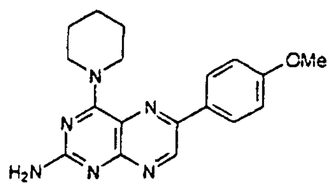 | 4.6 | 14 | 12 |
| 40 | 2-amino-4-piperidino-6-(3,4-dimethoxyphenyl)pteridine<br>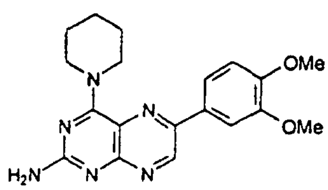 | 0.4 | 0.5 | 0.4 |
| 41 | 2-amino-4-N-methylpiperazino-6-phenylpteridine<br>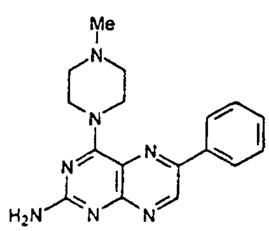 | 15 | 40 | 25 |

| | | | | |
|---|---|---|---|---|
| 42 | 2-amino-4-N-methylpiperazino-6-(4-chlorophenyl)pteridine<br>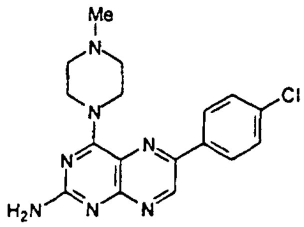 | 4.8 | 11 | 4.2 |
| 43 | 2-amino-4-N-methylpiperazino-6-(4-methoxyphenyl)pteridine<br>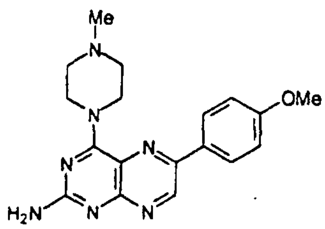 | 12.3 | 11.7 | 4.2 |
| 44 | 2-amino-4-methylpiperazino-6-(3,4-dimethoxyphenyl)pteridine<br>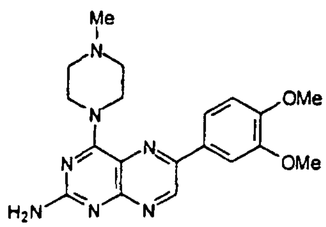 | 2 | 7.3 | 6.5 |
| 45 | 2-amino-4-cyclopentylamino-6-(4-methoxyphenyl)pteridine<br>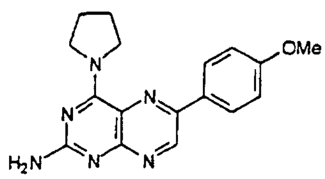 | 22 | 17 | 3.7 |
| 46 | 2-amino-4-piperazino-6-phenylpteridine<br>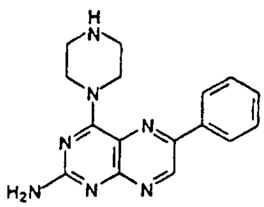 | 12 | 11.7 | 5 |

| | | | | |
|---|---|---|---|---|
| 47 | 2-amino-4-piperazino-6-(4-chlorophenyl)pteridine | | | |
| | 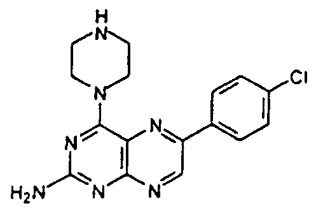 | 2 | 20 | 15 |
| 48 | 2-amino-4-piperazino-6-(4-methoxyphenyl)pteridine | | | |
| | 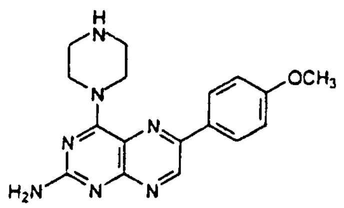 | 10.5 | 14 | 4.5 |
| 49 | 2-amino-4-piperazino-6-(3,4-dimethoxyphenyl)pteridine | | | |
| | 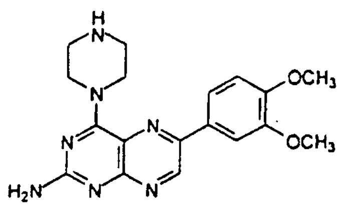 | 2.8 | 14.8 | 4.5 |
| 50 | 2-amino-4-dibenzylamino-6-(3,4,5-trimethoxyphenyl)pteridine | | | |
| | 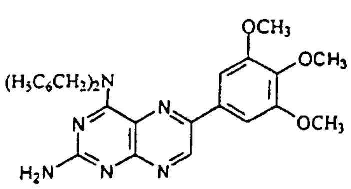 | ++ | ++ | ++ |

| | | | | |
|---|---|---|---|---|
| 51 | 2-amino-4-morpholino-6-(3,4,5-trimethoxyphenyl)pteridine<br>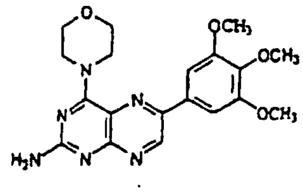 | ++ | + | + |
| 52 | 2-amino-4-adamantylamino-6-(3,4,5-trimethoxyphenyl)pteridine<br>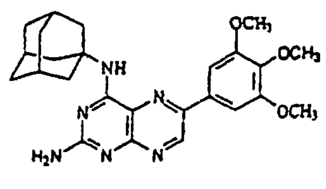 | ++ | ++ | ++ |
| 53 | 2-amino-4-adamantylamino-6-naftylpteridine<br>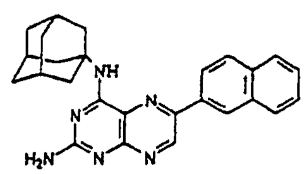 | 0 | + | + |
| 54 | 2-amino-4-adamantylamino-6-(3,4,5-trimethoxyphenyl)pteridine<br>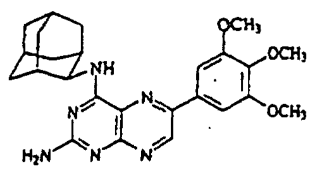 | +++ | ++ | ++ |
| 55 | 2-amino-4-adamantylamino-6-naftylpteridine<br>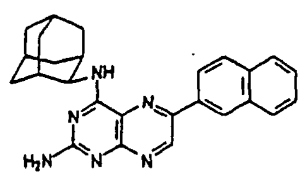 | ++ | ++ | ++ |

56 2-amino-4-morpholino-6-(3,4-formylidene-3,4-dihydroxyphenyl)pteridine

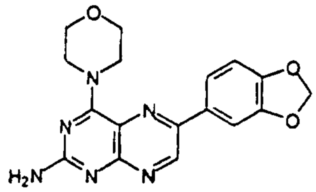

++ + +

57 2-amino-4-dimethylamino-6-(3,4-formylidene-3,4-dihydroxyphenyl)pteridine

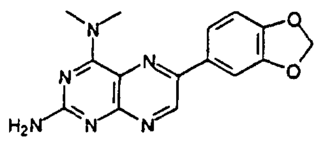

0 + +

58 2-amino-4-cyclopentylamino-6-(3,4-dimethoxyphenyl)pteridine

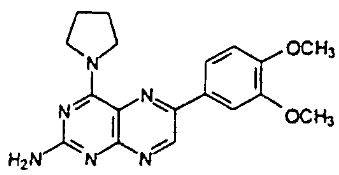

++ ++ +

59 2-amino-4-dimethylamino-6-(3,4-dimethoxyphenyl)pteridine

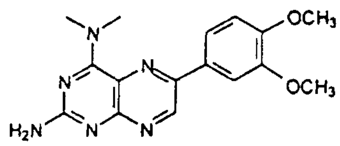

+++ ++ ++

60 2-amino-4-dimethylamino-6-methylpteridine

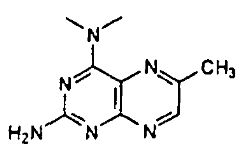

+ + +

| | | | | |
|---|---|---|---|---|
| 61 | 2-amino-4-ethoxy-6-phenylpteridine<br>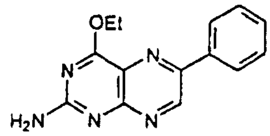 | ++ | ++ | ++ |
| 62 | 2-amino-4-propylamino-6-phenylpteridine<br>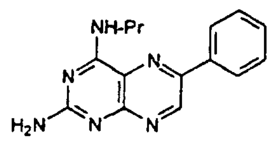 | ++ | + | + |
| 63 | 2-amino-4-propylamino-6-(3,4-dimethoxyphenyl)pteridine<br>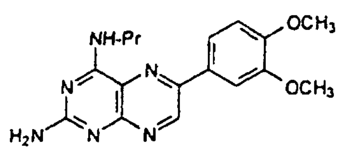 | +++ | ND | ND |
| 64 | 2-acetamido-4-hydroxy-6-(3,4-dimethoxyphenyl)pteridine<br>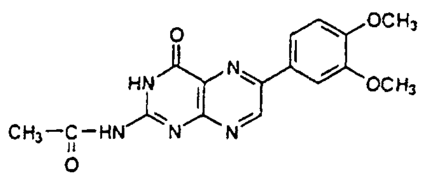 | + | 0 | + |
| 65 | 2-acetamido-4-i-propoxy-6-(3,4-dimethoxyphenyl)pteridine<br>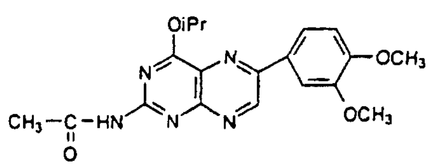 | ++ | + | + |

| | | | | |
|---|---|---|---|---|
| 66 | 2-amino-4-ethoxy-6-(3,4-dimethoxyphenyl)pteridine | | | |
| | 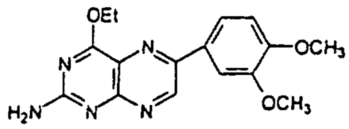 | ND | +++ | +++ |

Table I

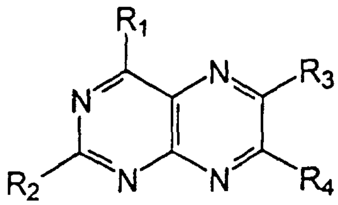

| n° | $R_1$ | $R_2$ | $R_3$ | $R_4$ |
|---|---|---|---|---|
| 1 | $OC_5H_{11}$ | $NH_2$ | 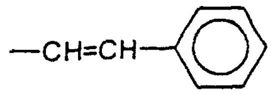 | H |
| 2 | $OC_5H_{11}$ | $NH_2$ | 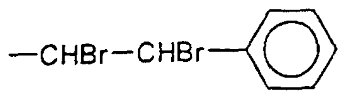 | H |
| 3 | $OC_5H_{11}$ | $NH_2$ | 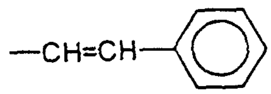 | $OCH_3$ |
| 4 | $OCH_3$ | $NH_2$ | $-CHOH-CHOH-CH_3$ | H |
| 5 | $NH_2$ | $NH_2$ | 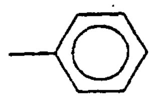 | H |
| 6 | $NMe_2$ | $NH_2$ | 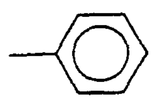 | $CH_3$ |
| 7 | $NH_2$ | $NH_2$ | 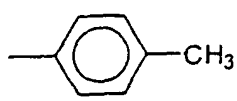 | H |
| 8 | $NH_2$ | $NH_2$ | 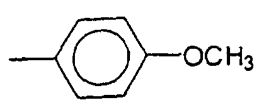 | H |
| 9 | $NH_2$ | $NH_2$ | 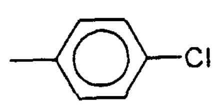 | H |
| 10 | $NH_2$ | $NHCH_2CH_2OH$ | 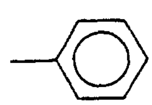 | H |

| | | | | |
|---|---|---|---|---|
| 11 | $NH_2$ | $NHCH_2CH_2OH$ | 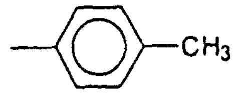 | H |
| 12 | $NH_2$ | NH-$CH_2CH_2OH$ | 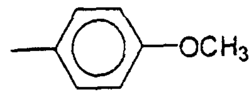 | H |
| 13 | NHOH | $NH_2$ | 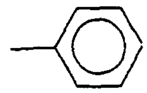 | H |
| 14 | $NMe_2$ | $NH_2$ | 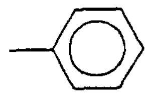 | H |
| 15 | $NMe_2$ | $NH_2$ | 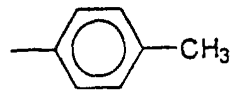 | H |
| 16 | $NMe_2$ | $NH_2$ | 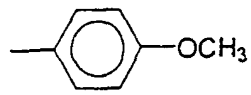 | H |
| 17 | $NH_2$ | $NH_2$ | $CH_2OCH_2CH_2OCH_3$ | H |
| 18 | $NH_2$ | $NH_2$ | $CH_2O(CH_2)_9CH_3$ | H |
| 19 | $NH_2$ | $NH_2$ | $CH_2NHCH_2$ 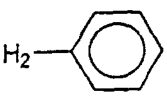 | H |
| 20 | $NH_2$ | $NH_2$ | $CH_2N(CH_3)_2$ | H |
| 21 | $NEt_2$ | $NH_2$ | 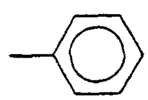 | H |
| 22 | $NEt_2$ | $NH_2$ | 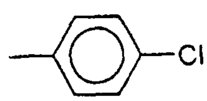 | H |
| 23 | $NEt_2$ | $NH_2$ | 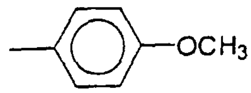 | H |

| | | | | |
|---|---|---|---|---|
| 24 | $NEt_2$ | $NH_2$ | 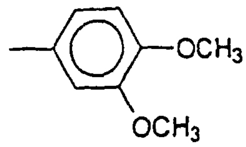 | H |
| 25 | 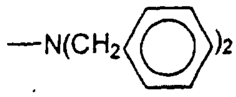 | $NH_2$ | 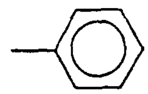 | H |
| 26 | 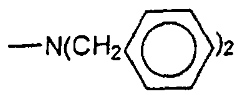 | $NH_2$ | 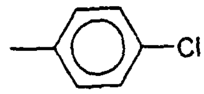 | H |
| 27 | 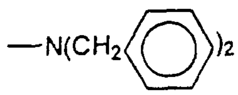 | $NH_2$ | 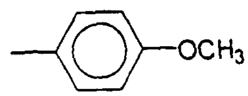 | H |
| 28 | 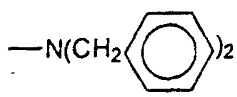 | $NH_2$ | 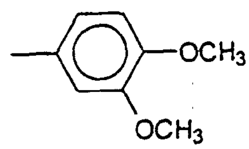 | H |
| 29 | $-N(CH_2CH_2CH_3)_2$ | $NH_2$ | 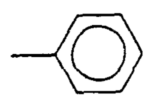 | H |
| 30 | $-N(CH_2CH_2CH_3)_2$ | $NH_2$ | 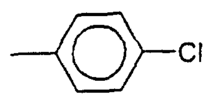 | H |
| 31 | $-N(CH_2CH_2CH_3)_2$ | $NH_2$ | 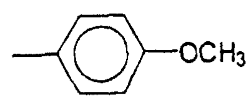 | H |
| 32 | $-N(CH_2CH_2CH_3)_2$ | $NH_2$ | 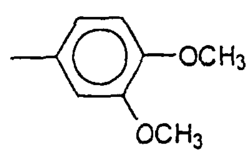 | H |
| 33 | 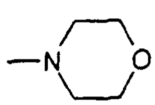 | $NH_2$ | 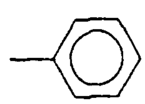 | H |

| | | | | |
|---|---|---|---|---|
| 34 | 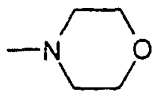 | $NH_2$ | 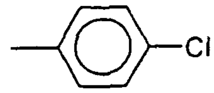 | H |
| 35 | 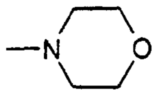 | $NH_2$ | 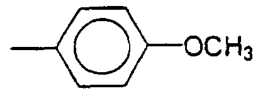 | H |
| 36 | 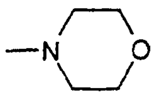 | $NH_2$ | 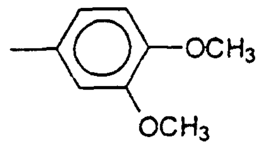 | H |
| 37 | 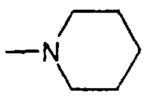 | $NH_2$ | 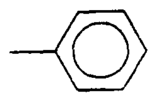 | H |
| 38 | 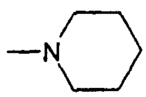 | $NH_2$ | 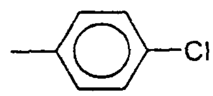 | H |
| 39 | 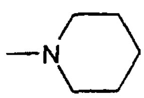 | $NH_2$ | 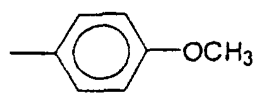 | H |
| 40 | 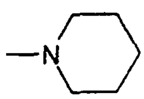 | $NH_2$ | 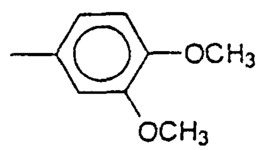 | H |
| 41 | 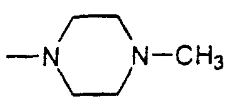 | $NH_2$ | 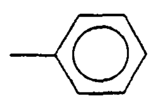 | H |
| 42 | 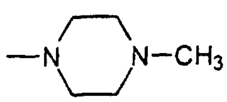 | $NH_2$ | 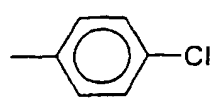 | H |
| 43 | 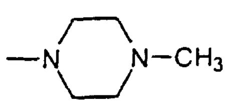 | $NH_2$ | 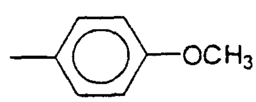 | H |

| | | | | |
|---|---|---|---|---|
| 44 | 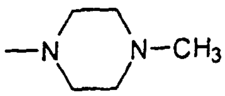 | $NH_2$ | 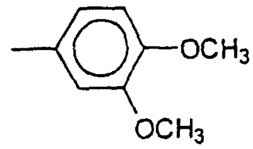 | H |
| 45 | 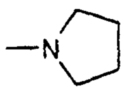 | $NH_2$ | 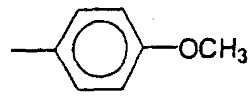 | H |
| 46 | 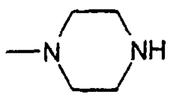 | $NH_2$ | 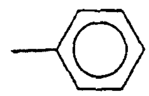 | H |
| 47 | 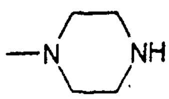 | $NH_2$ | 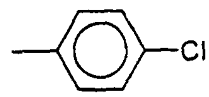 | H |
| 48 | 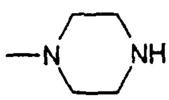 | $NH_2$ | 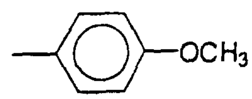 | H |
| 49 | 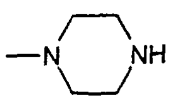 | $NH_2$ | 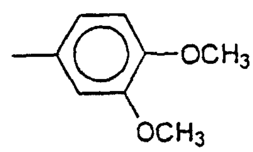 | H |

Table II

| IC$_{50}$ in µM of pteridine derivative | | | |
|---|---|---|---|
| Compound n° | MLR | ACD3 | aCD28 |
| 1 | 15 | 110 | 80 |
| 2 | 12 | 4 | 12 |
| 3 | 25 | 160 | 100 |
| 4 | >200 | 140 | 110 |
| 5 | >200 | >200 | >200 |
| 6 | >200 | >200 | >200 |
| 7 | >200 | 110 | 25 |
| 8 | 150 | 125 | 22 |
| 9 | 125 | 110 | 110 |
| 10 | 125 | 125 | 125 |
| 11 | >200 | 200 | 160 |

Table II (continued)

| IC$_{50}$ in μM of pteridine derivative | | | |
|---|---|---|---|
| Compound n° | MLR | ACD3 | aCD28 |
| 12 | >200 | >200 | 160 |
| 13 | 140 | >200 | >200 |
| 14 | 5.0 | 15 | 15 |
| 15 | 3.6 | 4.5 | 3.9 |
| 16 | 12 | 7.5 | 7.5 |
| 17 | >200 | 180 | 18 |
| 18 | >200 | 152 | 100 |
| 19 | 50 | 123 | 118 |
| 20 | >200 | 170 | 100 |
| 21 | 15 | 80 | 20 |
| 22 | 17 | 50 | 20 |
| 23 | 12 | 20 | 20 |
| 24 | 0.08 | 0.5 | 0.1 |
| 25 | 75 | 25 | 25 |
| 26 | 100 | 75 | 20 |
| 27 | 50 | 20 | 15 |
| 28 | 3.5 | 4.3 | 3.2 |
| 29 | 15 | 25 | 25 |
| 30 | 13 | 9.7 | 6.7 |
| 31 | 8.5 | 5.7 | 2.8 |
| 32 | 2.2 | 0.8 | 0.7 |
| 33 | 15 | 100 | 20 |
| 34 | 12 | 20 | 20 |
| 35 | 15 | 125 | 20 |
| 36 | 0.1 | 0.4 | 0.3 |
| 37 | 15 | 80 | 20 |
| 38 | 15 | 70 | 25 |
| 39 | 4.6 | 14 | 12 |
| 40 | 0.4 | 0.5 | 0.4 |
| 41 | 15 | 40 | 25 |
| 42 | 4.8 | 11 | 4.2 |
| 43 | 12.3 | 11.7 | 4.2 |
| 44 | 2 | 7.3 | 6.5 |
| 45 | 22 | 17 | 3.7 |
| 46 | 12 | 11.7 | 5 |
| 47 | 2 | 20 | 15 |
| 48 | 10.5 | 14 | 4.5 |
| 49 | 2.8 | 14.8 | 4.5 |

Table III

| I.S. | IC50 | | |
|---|---|---|---|
| Immunosuppressant | | | |
| | MLR | aCD3 | aCD28 |
| CyA | 20 nM | 50 nM | N.S. |
| FK506 | 1 nM | 1 nM | N.S. |
| Rapamycin | 1 nM | 1 nM | 1 nM |

Table III (continued)

| I.S. | IC50 | | |
|---|---|---|---|
| Immunosuppressant | | | |
| | MLR | aCD3 | aCD28 |
| Leflunomide | 25 µM | 15 µM | 20 µM |
| Mofetil | <0.5µM | 50 nM | 50 nM |
| MTX | 10 µM | >200 µM | > 200 µM |
| 5-FU | | 50µM | 17 µM |
| N.S. = not suppressive even not in the highest Concentration | | | |

Table IV

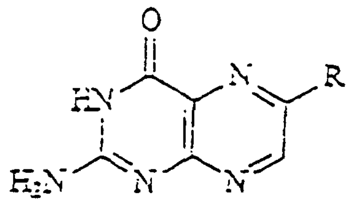

| R | MLR | aCD3 | aCD28 |
|---|---|---|---|
| $CH_2OOCOCH_3$ | 0 | 0 | 0 |
| $CH_2OC_2H_5$ | 0 | 0 | 0 |
| $CH_2NHCH_3$ | 0 | 0 | 0 |
| $CH_2N(CH_3)_2$ | 0 | 0 | 0 |
| $CH_2S\ CH_3$ | 0 | 0 | 0 |

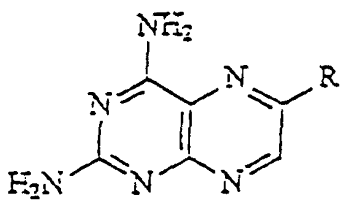

| R | MLR | aCD3 | aCD28 |
|---|---|---|---|
| $CH_2\ S\ CH_3$ | 0 | 0 | 0 |
| $CH_2\ O\ CH_2(CH_3)_2$ | 0 | 0 | 0 |
| $CH_2\ O\ CH_3$ | 0 | 0 | 0 |
| $CH_2\ O\ CH_2CH_2CH_3$ | 0 | 0 | 0 |
| $CH_2\ NH\ CO\ CH\ (CH_3)_2$ | 0 | 0 | 0 |

## Claims

1. Pharmaceutical composition for use in the treatment of autoimmuno disorders or for the treatment and/or prevention of transplant-rejections and/or for the treatment of inflammatory diseases comprising as an active principle at least one pteridine derivative having the general formula:

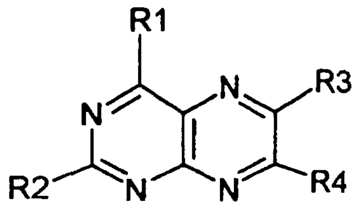

(I)

wherein:

$R_1$ is hydroxylamino, (mono- or di) $C_{1-7}$ alkylamino, (mono- or di) $C_{1-7}$ alkyloxyamino, (mono- or di) arylamino, (mono- or di) $C_{3-10}$ cycloalkylamino, (mono- or di) hydroxy $C_{1-7}$ alkylamino, (mono- or di) $C_{1-4}$ alkyl-arylamino, mercapto $C_{1-7}$ alkyl, $C_{1-7}$ alkyloxy or a saturated or unsaturated heterocyclic compound containing at least one nitrogen and optionally substituted by one or more $C_{1-4}$ alkyl, hydroxy $C_{1-4}$ alkyl, $C_{1-4}$ alkyloxy, halo, hydroxy, hydroxycarbonyl, $C_{1-4}$ alkyloxycarbonyl,

$R_2$ is amino, hydroxylamino, (mono- or di) $C_{1-7}$ alkylamino, (mono- or di) $C_{1-7}$ alkyloxyamino, (mono- or di) arylamino, (mono- or di) $C_{3-10}$ cycloalkylamino, (mono- or di) hydroxy $C_{1-7}$ alkylamino, (mono- or di) $C_{1-4}$ alkyl-arylamino, mercapto $C_{1-7}$ alkyl, $C_{1-7}$ alkyloxy or a saturated or unsaturated heterocyclic compound containing at least one nitrogen and optionally substituted by one or more $C_{1-4}$ alkyl, hydroxy $C_{1-4}$ alkyl, $C_{1-4}$ alkyloxy, halo, hydroxy, hydroxycarbonyl, $C_{1-4}$ alkyloxycarbonyl,

$R_3$ is an unsubstituted, monosubstituted or disubstituted aryl group (wherein the substituent may be, but not limited to, halogen, $C_{1-4}$ alkoxy, $C_{1-4}$ alkyl), aryl group bonded to the pteridine ring via a saturated or unsaturated aliphatic spacer which may be halogenated or hydroxylated, aliphatic substituent which may contain ether function, alcohol function, substituted or unsubstituted amino functions or $C_{1-4}$ alkyloxy,

$R_4$ is hydrogen, alkyl, alkoxy, substituted or unsubstituted aryl

or a pharmaceutical acceptable addition salt or a stereochemical isomeric form thereof.

**2.** Pharmaceutical composition according to claim 1, wherein $R_1$ is di($C_{1-4}$ alkyl) amino, morpholinyl, piperidinyl, piperazinyl, $C_{1-4}$ alkylpiperazinyl, pyrrolidinyl or benzylamine.

**3.** Pharmaceutical composition according to claim 1 or 2, wherein $R_2$ is ammonium, hydroxyammonium, (mono-or di) hydroxy $C_{1-7}$ alkylammonium.

**4.** Pharmaceutical composition according to claim 1, 2 or 3, wherein $R_3$ is benzyl, phenyl, styryl, phenyl-$C_{1-4}$ alkyloxy, phenyl ($C_{1-4}$ alkyl) optionally substituted by one or more $C_{1-4}$ alkyl or halo.

**5.** Pharmaceutical composition according to any of claims 1 to 4, wherein $R_4$ is hydrogen or $C_{1-4}$ alkyl.

**6.** Pharmaceutical composition according to any of claims 1 to 5, wherein the pteridine derivative is a compound chosen from the group comprising:

2-amino-4-pentoxy-6-styrylpteridine,
2-amino-4-n-pentoxy-6-(1,2-dibromo-2-phenylethyl) pteridine,
2-amino-4-methoxy-6-styryl-7-methoxypteridine,
2-amino-4-methoxy-6-(1,2-dihydroxypropyl) pteridine,
2-amino-4-hydroxylamino-6-phenylpteridine,
2-amino-4-dimethylamino-6-phenylpteridine,
2-amino-4-dimethylamino-6-(4-tolyl) pteridine,
2-amino-4-dimethylamino-6-(4-methoxyphenyl) pteridine,
2-amino-4-diethylamino-6-phenylpteridine,

2-amino-4-diethylamino-6-(4-chlorophenyl) pteridine,
2-amino-4-diethylamino-6-(4-methoxyphenyl) pteridine,
2-amino-4-diethylamino-6-(3,4-dimethoxyphenyl) pteridine,
2-amino-4-dibenzylamino-6-phenyl pteridine,
2-amino-dibenzylamino-6-(4-chlorophenyl) pteridine,
2-amino-4-dibenzylamino-6-(4-methoxyphenyl) pteridine,
2-amino-4-dibenzylamino-6-(3,4-dimethoxyphenyl) pteridine,
2-amino-4-dipropylamino-6-phenylpteridine,
2-amino-4-dipropylamino-6-(4-chlorophenyl) pteridine,
2-amino-4-dipropylamino-6-(4-methoxyphenyl) pteridine,
2-amino-4-dipropylamino-6-(3,4-dimethoxyphenyl) pteridine,
2-amino-4-morpholino-6-phenylpteridine,
2-amino-4-morpholino-6-(4-chlorophenyl) pteridine,
2-amino-4-morpholino-6-(4-methoxyphenyl) pteridine,
2-amino-4-morpholino-6-(3,4-dimethoxyphenyl) pteridine,
2-amino-4-piperidino-6-phenylpteridine,
2-amino-4-piperidino-6-(4-chlorophenyl) pteridine,
2-amino-4-piperidino-6-(4-methoxyphenyl) pteridine,
2-amino-4-piperidino-6-(3,4-dimethoxyphenyl) pteridine,
2-amino-4-N-methylpiperazino-6-phenylpteridine,
2-amino-4-N-methylpiperazino-6-(4-chlorophenyl) pteridine,
2-amino-4-N-methylpiperazino-6-(4-methoxyphenyl) pteridine,
2-amino-4-methylpiperazino-6-(3,4-dimethoxyphenyl) pteridine,
2-amino-4-cyclopentylamino-6-(4-methoxyphenyl) pteridine,
2-amino-4-piperazino-6-phenylpteridine,
2-amino-4-piperazino-6-(4-chlorophenyl) pteridine,
2-amino-4-piperazino-6-(4-methoxyphenyl) pteridine,
2-amino-4-piperazino-6-(3,4-dimethoxyphenyl) pteridine,
2-amino-4-dibenzylamino-6-(3,4,5-trimethoxyphenyl) pteridine,
2-amino-4-morpholino-6-(3,4,5-trimethoxyphenyl) pteridine,
2-amino-4-adamantylamino-6-(3,4,5-trimethoxyphenyl) pteridine,
2-amino-4-adamantylamino-6-naphtylpteridine,
2-amino-4-adamantylamino-6-(3,4,5-trimethoxyphenyl) pteridine,
2-amino-4-adamantylamino-6-naphtylpteridine,
2-amino-4-morpholino-6-(3,4-formylidene-3,4-dihydroxyphenyl) pteridine,
2-amino-4-dimethylamino-6-(3,4-formylidene-3,4-dihydroxyphenyl) pteridine,
2-amino-4-cyclopentylamino-6-(3,4-dimethoxyphenyl) pteridine,
2-amino-4-dimethylamino-6-(3,4-dimethoxyphenyl) pteridine,
2-amino-4-dimethylamino-6-methylpteridine,
2-amino-4-ethoxy-6-phenylpteridine,
2-amino-4-propylamino-6-phenylpteridine,
2-amino-4-propylamino-6-(3,4-dimethoxyphenyl) pteridine,
2-acetamido-4-hydroxy-6-(3,4-dimethoxyphenyl) pteridine,
2-acetamido-4-i-propoxy-6-(3,4-dimethoxyphenyl) pteridine, and
2-amino-4-ethoxy-6-(3,4-dimethoxyphenyl) pteridine.

**7.** Pharmaceutical composition according to any of claims 1 to 6, further comprising one or more other immunosuppressants.

**8.** Pharmaceutical composition according to claim 7, wherein the said one or more other immunosuppressants are chosen from the group comprising cyclosporine A, tacrolimus, rapamycine, leflunomide, mofetil, methylxanthines and 8-phenylxanthines.

**9.** Pharmaceutical composition according to any of claims 1 to 8, comprising as an active principle a therapeutically active non-toxic acid addition salt of a pteridine derivative having the general formula (I), wherein the said acid is selected from the group consisting of hydrohalic acids; sulfuric acid; nitric acid; phosphoric acid; organic acids such as acetic, propanoic, hydroxyacetic, 2-hydroxypropanoic, 2-oxopropanoic, ethanedioic, propanedioic, butanedioic, (Z)-2-butenedioic, (E)2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic and 4-amino-2-hydroxybenzoic acids.

**10.** Pharmaceutical composition according to any of claims 1 to 8, comprising as an active principle a therapeutically active non-toxic base addition salt form of a pteridine derivative having the general formula (I), wherein the said base addition salt form is selected from the group consisting of sodium, potassium and calcium salts; and salts with pharmaceutically acceptable amines such as ammonia, alkylamines, benzathine, N-methyl-D-glucamine and hydrabamine and amino-acids such as arginine and lysine.

**11.** Pharmaceutical composition according to any of claims 1 to 8, comprising as an active principle a solvate of a pteridine derivative having the general formula (I).

**12.** Pharmaceutical composition according to claim 11, wherein the said solvate is a hydrate or an alcoholate.

**13.** Compound having the general formula:

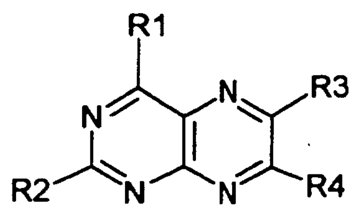

(I)

wherein:

$R_1$ is hydroxylamino, (mono- or di) $C_{1-7}$ alkylamino, (mono- or di) $C_{1-7}$ alkyloxyamino, (mono- or di) arylamino, (mono- or di) $C_{3-10}$ cycloalkylamino, (mono- or di) hydroxy $C_{1-7}$ alkylamino, (mono- or di) $C_{1-4}$ alkyl-arylamino, mercapto $C_{1-7}$ alkyl, $C_{1-7}$ alkyloxy or a saturated or unsaturated heterocyclic compound containing at least one nitrogen and optionally substituted by one or more $C_{1-4}$ alkyl, hydroxy $C_{1-4}$ alkyl, $C_{1-4}$ alkyloxy, halo, hydroxy, hydroxycarbonyl, $C_{1-4}$ alkyloxycarbonyl,

$R_2$ is amino, hydroxylamino, (mono- or di) $C_{1-7}$ alkylamino, (mono- or di) $C_{1-7}$ alkyloxyamino, (mono- or di) arylamino, (mono- or di) $C_{3-10}$ cycloatkylamino, (mono- or di) hydroxy $C_{1-7}$ alkylamino, (mono- or di) $C_{1-4}$ alkyl-arylamino, mercapto $C_{1-7}$ alkyl, $C_{1-7}$ alkyloxy or a saturated or unsaturated heterocyclic compound containing at least one nitrogen and optionally substituted by one or more $C_{1-4}$ alkyl, hydroxy $C_{1-4}$ alkyl, $C_{1-4}$ alkyloxy, halo, hydroxy, hydroxycarbonyl, $C_{1-4}$ alkyloxycarbonyl,

$R_3$ is an unsubstituted, monosubstituted or disubstituted aryl group (wherein the substituent may be, but not limited to, halogen, $C_{1-4}$ alkoxy, $C_{1-4}$ alkyl), aryl group bonded to the pteridine ring via a saturated or unsaturated aliphatic spacer which may be halogenated or hydroxylated, aliphatic substituent which may contain ether function, alcohol function, substituted or unsubstituted amino functions or $C_{1-4}$ alkyloxy,

$R_4$ is hydrogen, alkyl, alkoxy, substituted or unsubstituted aryl,

or a pharmaceutical acceptable addition salt or a stereochemical isomeric form thereof,
with the exception of:

- 2-amino-4-methylamino-6-phenyl-7-phenylpteridine,
- 2-amino-4-dimethylamino-6-phenyl-7-phenylpteridine,
- 2-methylamino-4-methylamino-6-methyl-7-methylpteridine,
- 2-methylamino-4-methylamino-6-phenylpteridine,
- 2-methylamino-4-methylamino-6-phenyl-7-phenylpteridine,
- 2-methylamino-4-methylamino-6-(*o*-chlorophenyl)-7-(*o*-chlorophenyl)pteridine,
- 2-methylamino-4-methylamino-6-(*m*-chlorophenyl)-7-(*m*-chlorophenyl)pteridine,
- 2-methylamino-4-methylamino-6-(*p*-chlorophenyl)-7-(*p*-chlorophenyl)pteridine,

- 2-methylamino-4-methylamino-6-(*p*-methoxyphenyl)-7-(*p*-methoxyphenyl)pteridine,
- 2-methylamino-4-dimethylamino-6-phenyl-7-phenylpteridine,
- 2-ethylamino-4-methylamino-6-phenyl-7-phenylpteridine,
- 2-dimethylamino-4-ethoxy-6-phenylpteridine,
- 2-dimethylamino-4-methylamino-6-phenyl-7-phenylpteridine,
- 2-dimethylamino-4-methylamino-6-phenyl-7-(*p*-chlorophenyl)pteridine,
- 2-dimethylamino-4-dimethylamino-6-propyl-7-propylpteridine,
- 2-dimethylamino-4-dimethylamino-6-phenylpteridine,
- 2-dimethylamino-4-dimethylamino-6-phenyl-7-phenylpteridine,
- 2-dimethylamino-4-piperidino-6-phenyl-7-phenylpteridine,
- 2-dimethylamino-4-morpholino-6-phenyl-7-phenylpteridine,
- 2-amino-4-ethoxy-6,7-diphenylpteridine,
- 2-amino-4-diethylamino-6,7-diphenylpteridine, and
- 2-amino-4-piperidino-6,7-diphenylpteridine.

**14.** Compound according to claim 13, wherein $R_1$ is di($C_{1-4}$ alkyl) amino, morpholinyl, piperidinyl, piperazinyl, $C_{1-4}$ alkylpiperazinyl, pyrrolidinyl or benzylamine.

**15.** Compound according to claim 13 or claim 14, wherein $R_2$ is ammonium, hydroxyammonium, (mono-or di) hydroxy $C_{1-7}$ alkylammonium.

**16.** Compound according to any of claims 13 to 15, wherein $R_3$ is benzyl, phenyl, styryl, phenyl-$C_{1-4}$ alkyloxy, phenyl ($C_{1-4}$ alkyl) optionally substituted by one or more $C_{1-4}$ alkyl or halo.

**17.** Compound according to any of claims 13 to 16, wherein $R_4$ is hydrogen or $C_{1-4}$ alkyl.

**18.** Compound according to claim 13, being chosen from the group comprising:

2-amino-4-pentoxy-6-styrylpteridine,
2-amino-4-n-pentoxy-6-(1,2-dibromo-2-phenylethyl) pteridine,
2-amino-4-methoxy-6-styryl-7-methoxypteridine,
2-amino-4-dimethylamino-6-phenylpteridine,
2-amino-4-dimethylamino-6-(4-tolyl) pteridine,
2-amino-4-dimethylamino-6-(4-methoxyphenyl) pteridine,
2-amino-4-diethylamino-6-phenylpteridine,
2-amino-4-diethylamino-6-(4-chlorophenyl) pteridine,
2-amino-4-diethylamino-6-(4-methoxyphenyl) pteridine,
2-amino-4-diethylamino-6-(3,4-dimethoxyphenyl) pteridine,
2-amino-4-dibenzylamino-6-phenyl pteridine,
2-amino-dibenzylamino-6-(4-chlorophenyl) pteridine,
2-amino-4-dibenzylamino-6-(4-methoxyphenyl) pteridine,
2-amino-4-dibenzylamino-6-(3,4-dimethoxyphenyl) pteridine,
2-amino-4-dipropylamino-6-phenylpteridine,
2-amino-4-dipropylamino-6-(4-chlorophenyl) pteridine,
2-amino-4-dipropylamino-6-(4-methoxyphenyl) pteridine,
2-amino-4-dipropylamino-6-(3,4-dimethoxyphenyl) pteridine,
2-amino-4-morpholino-6-phenylpteridine,
2-amino-4-morpholino-6-(4-chlorophenyl) pteridine,
2-amino-4-morpholino-6-(4-methoxyphenyl) pteridine,
2-amino-4-morpholino-6-(3,4-dimethoxyphenyl) pteridine,
2-amino-4-piperidino-6-phenylpteridine,
2-amino-4-piperidino-6-(4-chlorophenyl) pteridine,
2-amino-4-piperidino-6-(4-methoxyphenyl) pteridine,
2-amino-4-piperidino-6-(3,4-dimethoxyphenyl) pteridine,
2-amino-4-N-methylpiperazino-6-phenylpteridine,
2-arnino-4-N-methylpiperazino-6-(4-chlorophenyl) pteridine,
2-amino-4-N-methylpiperazino-6-(4-methoxyphenyl) pteridine,
2-amino-4-methylpiperazino-6-(3,4-dimethoxyphenyl) pteridine,

2-amino-4-cyclopentylamino-6-(4-methoxyphenyl) pteridine,
2-amino-4-piperazino-6-phenylpteridine,
2-amino-4-piperazino-6-(4-chlorophenyl) pteridine,
2-amino-4-piperazino-6-(4-methoxyphenyl) pteridine,
2-amino-4-piperazino-6-(3,4-dimethoxyphenyl) pteridine,
2-amino-4-dibenzylamino-6-(3,4,5-trimethoxyphenyl) pteridine,
2-amino-4-morpholino-6-(3,4,5-trimethoxyphenyl) pteridine,
2-amino-4-adamantylamino-6-(3,4,5-trimethoxyphenyl) pteridine,
2-amino-4-adamantylamino-6-naphtylpteridine,
2-amino-4-adamantylamino-6-(3,4,5-trimethoxyphenyl) pteridine,
2-amino-4-adamantylamino-6-naphtylpteridine,
2-amino-4-morpholino-6-(3,4-formylidene-3,4-dihydroxyphenyl) pteridine,
2-amino-4-dimethylamino-6-(3,4-formylidene-3,4-dihydroxyphenyl) pteridine,
2-amino-4-cyclopentylamino-6-(3,4-dimethoxyphenyl) pteridine,
2-amino-4-dimethylamino-6-(3,4-dimethoxyphenyl) pteridine,
2-amino-4-dimethylamino-6-methylpteridine,
2-amino-4-ethoxy-6-phenylpteridine,
2-amino-4-propylamino-6-phenylpteridine,
2-amino-4-propylamino-6-(3,4-dimethoxyphenyl) pteridine,
2-acetamido-4-hydroxy-6-(3,4-dimethoxyphenyl) pteridine,
2-acetamido-4-i-propoxy-6-(3,4-dimethoxyphenyl) pteridine, and
2-amino-4-ethoxy-6-(3,4-dimethoxyphenyl) pteridine.

**19.** Compound according to claim 13, being the acid addition salt of a pteridine derivative having the general formula (I), wherein the said acid is selected from the group consisting of hydrohalic acids; sulfuric acid; nitric acid; phosphoric acid; organic acids such as acetic, propanoic, hydroxyacetic, 2-hydroxypropanoic, 2-oxopropanoic, ethanedioic, propanedioic, butanedioic, (Z)-2-butenedioic, (E)2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic and 4-amino-2-hydroxybenzoic acids.

**20.** Compound according to claim 13, being the base addition salt form of a pteridine derivative having the general formula (I), wherein the said base addition salt form is selected from the group consisting of sodium, potassium and calcium salts; and salts with pharmaceutically acceptable amines such as ammonia, alkylamines, benzathine, N-methyl-D-glucamine and hydrabamine and amino-acids such as arginine and lysine.

**21.** Compound according to claim 13, being a solvate of a pteridine derivative having the general formula (I).

**22.** Compound according to claim 21, wherein said solvate is a hydrate or an alcoholate.

**23.** Use of a compound according to any of claims 13 to 22 for the manufacture of a medicament for the treatment of autoimmune disorders.

**24.** Use of a compound according to any of claims 13 to 22 for the manufacture of a medicament for the prevention of transplant-rejections.

**25.** Use of a compound according to any of claims 13 to 22 for the manufacture of a medicament for the treatment of inflammatory diseases.

**26.** Method for selecting immunosuppressive agents from compounds according to any of claims 13 to 22 by a combination of at least three test systems based on MLR, aCD3 and aCD28.

**27.** Pharmaceutical composition comprising a compound according to any of claims 13 to 22 and a pharmaceutically acceptable carrier.

**Patentansprüche**

**1.** Pharmazeutische Zusammensetzung zur Verwendung in der Behandlung von Autoimmunkrankheiten oder zur

Behandlung und/oder Vorbeugung von Transplantatabstoßungen und/oder zur Behandlung von entzündlichen Erkrankungen, umfassend als Wirkprinzip zumindest ein Pteridin-Derivat mit der allgemeinen Formel:

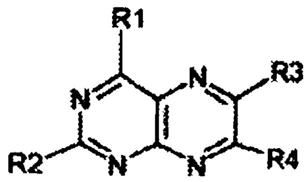

(I)

wobei:

$R_1$ Hydroxylamino, (Mono- oder Di)-$C_{1-7}$-Alkylamino, (Mono- oder Di)-$C_{1-7}$-Alkoxyamino, (Mono- oder Di)-Arylamino, (Mono- oder Di)-$C_{3-10}$-Cycloalkylamino, (Mono- oder Di)-Hydroxy-$C_{1-7}$-Alkylamino, (Mono- oder Di) -$C_{1-4}$-Alkyl-arylamino, Mercapto-$C_{1-7}$-Alkyl, $C_{1-7}$-Alkoxy oder eine gesättigte oder ungesättigte heterozyklische Verbindung ist, die zumindest ein Stickstoff enthält und wahlweise durch ein oder mehreres von $C_{1-4}$-Alkyl, Hydroxy-$C_{1-4}$-Alkyl, $C_{1-4}$-Alkyloxy, Halo, Hydroxy, Hydroxycarbonyl, $C_{1-4}$-Alkyloxycarbonyl substituiert ist,

$R_2$ Amino, Hydroxylamino, (Mono- oder Di) $C_{1-7}$-Alkylamino, (Mono- oder Di)-$C_{1-7}$-Alkyloxyamino, (Mono- oder Di)-Arylamino, (Mono- oder Di)-$C_{3-10}$-Cycloalkylamino, (Mono- oder Di)-Hydroxy-$C_{1-7}$-Alkylamino, (Mono- oder Di)-$C_{1-4}$-Alkyl-Arylamino, Mercapto-$C_{1-7}$-Alkyl, $C_{1-7}$-Alkyloxy oder eine gesättigte oder ungesättigte heterozyklische Verbindung ist, die zumindest ein Stickstoffatom enthält und wahlweise durch ein oder mehr von $C_{1-4}$-Alkyl, Hydroxy-$C_{1-4}$-Alkyl, $C_{1-4}$-Alkyloxy, Halo, Hydroxy, Hydroxy-carbonyl, $C_{1-4}$-Alkyloxycarbonyl substituiert ist,

$R_3$ eine unsubstituierte, monosubstituierte oder disubstituierte Arylgruppe (wobei der Substituent Halogen, $C_{1-4}$-Alkoxy, $C_{1-4}$-Alkyl sein kann, jedoch nicht hierauf beschränkt ist), eine Arylgruppe, gebunden an den Pteridin-Ring über einen gesättigten oder ungesättigten aliphatischen Spacer, der halogeniert oder hydroxyliert sein kann, ein aliphatischer Substituent ist, der eine Etherfunktion, Alkoholfunktion, substituierte oder unsubstituierte Aminofunktionen oder $C_{1-4}$-Alkyloxy enthalten kann,

$R_4$ Wasserstoff, Alkyl, Alkoxy, substituiertes oder unsubstituiertes Aryl ist, oder

ein pharmazeutisch verträgliches Additionssalz oder eine stereochemisch isomere Form hiervon.

2. Pharmazeutische Zusammensetzung nach Anspruch 1, wobei $R_1$ Di-($C_{1-4}$-Alkyl)-Amino, Morpholinyl, Piperidinyl, Piperazinyl, $C_{1-4}$-Alkylpiperazinyl, Pyrrolidinyl oder Benzylamin ist.

3. Pharmazeutische Zusammensetzung nach Anspruch 1 oder 2, wobei $R_2$ Ammonium, Hydroxylammonium, (Mono- oder Di)-Hydroxy-$C_{1-7}$-Alkylammonium ist.

4. Pharmazeutische Zusammensetzung nach Anspruch 1, 2 oder 3, wobei $R_3$ Benzyl, Phenyl, Styryl, Phenyl-$C_{1-4}$-Alkyloxy, Phenyl ($C_{1-4}$-Alkyl), wahlweise durch ein oder mehreres von $C_{1-4}$-Alkyl oder Halo substituiert, ist.

5. Pharmazeutische Zusammensetzung nach einem der Ansprüche 1 bis 4, wobei $R_4$ Wasserstoff oder $C_{1-4}$-Alkyl ist.

6. Pharmazeutische Zusammensetzung nach einem der Ansprüche 1 bis 5, wobei das Pteridin-Derivat eine Verbindung ist, ausgewählt aus der Gruppe, die folgendes umfasst:

2-Amino-4-pentoxy-6-styrylpteridin,
2-Amino-4-n-pentoxy-6-(1,2-dibromo-2-phenylethyl) pteridin,
2-Amino-4-methoxy-6-styryl-7-methoxypteridin,
2-Amino-4-methoxy-6-(1,2-dihydroxypropyl)pteridin,

2-Amino-4-hydroxylamino-6-phenylpteridin,
2-Amino-4-dimethylamino-6-phenylpteridin,
2-Amino-4-dimethylamino-6-(4-tolyl)pteridin,
2-Amino-4-dimethylamino-6-(4-methoxyphenyl)pteridin,
2-Amino-4-dimethylamino-6-phenylpteridin,
2-Amino-4-diethylamino-6-(4-chlorophenyl)pteridin,
2-Amino-4-diethylamino-6-(4-methoxyphenyl)pteridin,
2-Amino-4-diethylamino-6-(3,4-dimethoxyphenyl)pteridin,
2-Amino-4-dibenzylamino-6-phenylpteridin,
2-Amino-dibenzylamino-6-(4-chlorophenyl)pteridin,
2-Amino-4-dibenzylamino-6-(4-methoxyphenyl)pteridin,
2-Amino-4-dibenzylamino-6-(3,4-Dimethoxyphenyl)pteridin,
2-Amino-4-dipropylamino-6-phenylpteridin,
2-Amino-4-dipropylamino-6-(4-chlorophenyl)pteridin,
2-Amino-4-dipropylamino-6-(4-methoxyphenyl)pteridin,
2-Amino-4-dipropylamino-6-(3,4-dimethoxyphenyl)pteridin,
2-Amino-4-morpholino-6-phenylpteridin,
2-Amino-4-morpholino-6-(4-chlorphenyl)pteridin,
2-Amino-4-morpholino-6-(4-methoxyphenyl)pteridin,
2-Amino-4-morpholino-6-(3,4-dimethoxyphenyl)pteridin,
2-Amino-4-piperidino-6-phenylpteridin,
2-Amino-4-piperidino-6-(4-chlorophenyl)pteridin,
2-Amino-4-piperidino-6-(4-methoxyphenyl)pteridin,
2-Amino-4-piperidino-6-(3,4-dimethoxyphenyl)pteridin,
2-Amino-4-N-methylpiperazino-6-phenylpteridin,
2-Amino-4-N-methylpiperazino-6-(4-chlorophenyl)pteridin,
2-Amino-4-N-methylpiperazino-6-(4-methoxyphenyl)pteridin,
2-Amino-4-methylpiperazino-6-(3,4-dimethoxyphenyl)pteridin,
2-Amino-4-cyclopentylamino-6-(4-methoxyphenyl)pteridin,
2-Amino-4-piperazino-6-phenylpteridin,
2-Amino-4-piperazino-6-(4-chlorophenyl)pteridin,
2-Amino-4-piperazino-6-(4-methoxyphenyl)pteridin,
2-Amino-4-piperazino-6-(3,4-dimethoxyphenyl)pteridin,
2-Amino-4-dibenzylamino-6-(3,4,5-trimethoxyphenyl)pteridin,
2-Amino-4-morpholino-6-(3,4,5-trimethoxyphenyl)pteridin,
2-Amino-4-adamantylamino-6-(3,4,5-trimethoxyphenyl)pteridin,
2-Amino-4-adamantylamino-6-naphtylpteridin,
2-Amino-4-adamantylamino-6-(3,4,5-trimethoxyphenyl)pteridin,
2-Amino-4-adamantylamino-6-naphtylpteridin,
2-Amino-4-morpholino-6-(3,4-formyliden-3,4-dihydroxyphenyl)pteridin,
2-Amino-4-dimethylamino-6-(3,4-formyliden-3,4-dihydroxyphenyl)pteridin,
2-Amino-4-cyclopentylamino-6-(3,4-dimethoxyphenyl)pteridin,
2-Amino-4-dimethylamino-6-(3,4-dimethoxyphenyl)pteridin,
2-Amino-4-dimethylamino-6-methylpteridin,
2-Amino-4-ethoxy-6-phenylpteridin,
2-Amino-4-propylamino-6-phenylpteridin,
2-Amino-4-propylamino-6-(3,4-dimethoxyphenyl)pteridin,
2-Acetamido-4-hydroxy-6-(3,4-dimethoxyphenyl)pteridin,
2-Acetamido-4-i-propoxy-6-(3,4-dimethoxyphenyl)pteridin und
2-Amino-4-ethoxy-6-(3,4-dimethoxyphenyl)pteridin,

**7.** Pharmazeutische Zusammensetzung nach einem der Ansprüche 1 bis 6, die weiterhin ein oder mehrere weitere immunsuppressive Stoffe umfasst.

**8.** Pharmazeutische Zusammensetzung nach Anspruch 7, wobei die ein oder mehreren weiteren immunsuppressiven Stoffe aus der Gruppe ausgewählt sind, die Cyclosporin A, Tacrolimus, Rapamycin, Leflunomid, Mofetil, Methylxanthine und 8-Phenylxanthine umfasst.

**9.** Pharmazeutische Zusammensetzung nach einem der Ansprüche 1 bis 8, die als Wirkprinzip ein therapeutisch wirksames untoxisches Säureadditionssalz eines Pteridin-Derivats mit der allgemeinen Formel (1) umfasst, wobei die Säure aus der Gruppe ausgewählt ist, bestehend aus Halogenwasserstoffsäuren; Schwefelsäure, Salpetersäure, Phosphorsäure, organische Säuren beispielsweise Essigsäure, Propansäure, Hydroxyessigsäure, 2-Hydroxypropansäure, 2-Oxopropansäure, Ethandisäure, Propandisäure, Butandisäure, (Z)-2-Butendisäure, (E)-2-Butendisäure, 2-Hydroxybutandisäure, 2,3-Dihydroxy-butandisäure, 2-Hydroxy-1,2,3-Propantricarbonsäure, Methansulfonsäure, Ethansulfonsäure, Benzolsulfonsäure, 4-Methylbenzolsulfonsäure, Cyclohexansulfaminsäure, 2-Hydroxybenzoesäure und 4-Amino-2-Hydroxybenzoesäuren.

**10.** Pharmazeutische Zusammensetzung nach einem der Ansprüche 1 bis 8, die als Wirkprinzip eine therapeutisch wirksame untoxische basische Additionssalzform eines Pteridin-Derivates mit der allgemeinen Formel (1) aufweist, wobei die Basenadditionssalz-Form aus der Gruppe ausgewählt ist, die aus Natrium-, Kalium- und Kalziumsalzen besteht; und Salzen mit pharmazeutisch verträglichen Aminen, beispielsweise Ammoniak, Alkylaminen, Benzathin, N-Methyl-D-glucamin und Hydrabamin und Aminosäuren beispielsweise Arginin und Lysin.

**11.** Pharmazeutische Zusammensetzung nach einem der Ansprüche 1 bis 8, die als Wirkprinzip ein Solvat eines Pteridin-Derivates mit der allgemeinen Formel (1) umfasst.

**12.** Pharmazeutische Zusammensetzung nach Anspruch 11, wobei das Solvat ein Hydrat oder ein Alkoholat ist.

**13.** Verbindung mit der allgemeinen Formel,

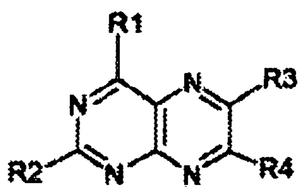

(I)

wobei:

$R_1$ Hydroxylamino, (Mono- oder Di) $C_{1-7}$-Alkylamino, (Mono- oder Di)-$C_{1-7}$-Alkyloxyamino, (Mono- oder Di) -Arylamino, (Mono- oder Di)-$C_{3-10}$-Cycloalkylamino, (Mono- oder Di) Hydroxy-$C_{1-7}$-Alkylamino, (Mono- oder Di)-$C_{1-4}$-Alkyl-Arylamino, Mercapto-$C_{1-7}$-Alkyl, $C_{1-7}$-Alkyloxy oder eine gesättigte oder ungesättigte heterozyklische Verbindung ist, die zumindest ein Stickstoffatom enthält und wahlweise durch ein oder mehreres von $C_{1-4}$-Alkyl, Hydroxy-$C_{1-4}$-Alkyl, $C_{1-4}$-Alkyloxy, Halo, Hydroxy, Hydroxycarbonyl, $C_{1-4}$-Alkyloxycarbonyl substituiert ist,

$R_2$ Amino, Hydroxylamino, (Mono- oder Di)-$C_{1-7}$-Alkylamino, (Mono- oder Di)-$C_{1-7}$-Alkyloxyamino, (Mono-oder Di)-Arylamino, (Mono- oder Di)-$C_{3-10}$-Cycloalkylamino, (Mono- oder Di)-Hydroxy $C_{1-7}$-Alkylamino, (Mono-oder Di-)-$C_{1-4}$-Alkyl-Arylamino, Mercapto-$C_{1-7}$-Alkyl, $C_{1-7}$-Alkyloxy oder eine gesättigte oder ungesättigte heterozyklische Verbindung ist, die zumindest ein Stickstoff enthält und wahlweise durch ein oder mehreres von $C_{1-4}$-Alkyl, Hydroxy $C_{1-4}$-Alkyl, $C_{1-4}$-Alkyloxy, Halo, Hydroxy, Hydroxycarbonyl, $C_{1-4}$-Alkyloxycarbonyl substituiert ist,

$R_3$ eine unsubstituierte, monosubstituierte oder disubstituierte Arylgruppe (wobei der Substituent Halogen, $C_{1-4}$-Alkoxy, $C_{1-4}$-Alkyl sein kann, jedoch hierauf nicht beschränkt ist), eine Arylgruppe, gebunden an den Pteridin-Ring über einen gesättigten oder ungesättigten aliphatischen Spacer, der halogeniert oder hydroxyliert sein kann, ein aliphatischer Substituent ist, der eine Etherfunktion, eine Alkoholfunktion, eine substituierte oder unsubstituierte Aminofunktionen oder $C_{1-4}$-Alkyloxy enthalten kann,

$R_4$ Wasserstoff, Alkyl, Alkoxy, substituiertes oder unsubstituiertes Aryl ist,

oder ein pharmazeutisch akzeptables Additionssalz oder eine stereochemisch isomere Form hiervon, mit der Ausnahme von:

- 2-Amino-4-methylamino-6-phenyl-7-phenylpteridin,
- 2-Amino-4-dimethylamino-6-phenyl-7-phenylpteridin,
- 2-Methylamino-4-methylamino-6-methyl-7-methylpteridin,
- 2-Methylamino-4-methylamino-6-phenylpteridin,
- 2-Methylamino-4-methylamino-6-phenyl-7-phenylpteridin,
- 2-Methylamino-4-methylamino-6-(o-chlorophenyl)-7-(o-chlorophenyl)pteridin,
- 2-Methylamino-4-methylamino-6-(m-chlorophenyl)-7-(m-chlorophenyl)pteridin,
- 2-Methylamino-4-methylamino-6-(p-chlorophenyl)-7-(p-chlorophenyl)pteridin,
- 2-Methylamino-4-methylamino-6-(p-methoxyphenyl)-7-(p-methoxyphenyl)pteridin,
- 2-Methylamino-4-dimethylamino-6-phenyl-7-phenylpteridin,
- 2-Ethylamino-4-methylamino-6-phenyl-7-phenylpteridin,
- 2-Dimethylamino-4-ethoxy-6-phenylpteridin,
- 2-Dimethylamino-4-methylamino-6-phenyl-7-phenylpteridin,
- 2-Dimethylamino-4-methylamino-6-phenyl-7-(pchlorophenyl)pteridin,
- 2-Dimethylamino-4-dimethylamino-6-propyl-7-propylpteridin,
- 2-Dimethylamino-4-dimethylamino-6-phenylpteridin,
- 2-Dimethylamino-4-dimethylamino-6-phenyl-7-phenylpteridin,
- 2-Dimethylamino-4-piperidino-6-phenyl-7-phenylpteridin,
- 2-Dimethylamino-4-morpholino-6-phenyl-7-phenylpteridin,
- 2-Amino-4-ethoxy-6,7-diphenylpteridin,
- 2-Amino-4-diethylamino-6,7-diphenylpteridin, und
- 2-Amino-4-piperidino-6,7-diphenylpteridin.

**14.** Verbindung nach Anspruch 13, wobei $R_1$ Di($C_{1-4}$-Alkyl)-Amino, Morpholinyl, Piperidinyl, Piperazinyl, $C_{1-4}$-Alkylpiperazinyl, Pyrrolidinyl oder Benzylamin ist.

**15.** Verbindung nach Anspruch 13 oder Anspruch 14, wobei $R_2$ Ammonium, Hydroxylammonium, (Mono- oder Di) -Hydroxy-$C_{1-7}$-Alkylammonium ist.

**16.** Verbindung nach einem der Ansprüche 13 bis 15, wobei $R_3$ Benzyl, Phenyl, Styryl, Phenyl-$C_{1-4}$-Alkyloxy, Phenyl-($C_{1-4}$-Alkyl), wahlweise substituiert von einem oder mehrerem von $C_{1-4}$-Alkyl oder Halo ist.

**17.** Verbindung nach Anspruch 13 bis 16, wobei $R_4$ Wasserstoff oder $C_{1-4}$-Alkyl ist.

**18.** Verbindung nach Anspruch 13, ausgewählt aus der Gruppe, die folgendes umfasst:

2-Amino-4-pentoxy-6-styrylpteridin,
2-Amino-4-n-pentoxy-6-(1,2-dibromo-2-phenylethyl)pteridin,
2-Amino-4-methoxy-6-styryl-7-methoxypteridin,
2-Amino-4-dimethylamino-6-phenylpteridin,
2-Amino-4-dimethylamino-6-(4-tolyl)pteridin,
2-Amino-4-dimethylamino-6-(4-methoxyphenyl)pteridin,
2-Arnino-4-dimethylamino-6-phenylpteridin,
2-Amino-4-diethylamino-6-(4-chlorophenyl)pteridin,
2-Amino-4-dimethylamino-6-(4-methoxyphenyl)pteridin,
2-Amino-4-dimethylamino-6-(3,4-dimethoxyphenyl)pteridin,
2-Amino-4-dibenzylamino-6-phenylpteridin,
2-Amino-dibenzylamino-6-(4-chlorophenyl)pteridin,
2-Amino-4-dibenaylamino-6-(4-methoxyphenyl)pteridin,
2-Amino-4-dibeznylamino-6-(3,4-dimethoxyphenyl)pteridin,
2-Amino-4-dipropylamino-6-phenylpteridin,
2-Amino-4-dipropylamino-6-(4-chlorophenyl)pteridin,
2-Amino-4-dipropylamino-6-(4-methoxyphenyl)pteridin,
2-Amino-4-dipropylamino-6-(3,4-dimethoxyphenyl)pteridin,
2-Amino-4-dipropylamino-6-(3,4-dimethoxyphenyl)pteridin,
2-Amino-4-morpholino-6-phenylpteridin,
2-Amino-4-morpholino-6-(4-chlorophenyl)pteridin,
2-Amino-4-morpholino-6-(methoxyphenyl)pteridin,

2-Amino-4-morpholino-6-(3,4-dimethoxyphenyl)pteridin,
2-Amino-4-piperidino-6-phenylpteridin,
2-Amino-4-piperidino-6-(4-chlorophenyl)pteridin,
2-Amino-4-piperidino-6-(4-methoxyphenyl)pteridin,
2-Amino-4-piperidino-6-(3,4-dimethoxyphenyl)pteridin,
2-Amino-4-N-methylpiperazino-6-phenylpteridin,
2-Amino-4-N-methylpiperazino-6-(4-chlorophenyl)pteridin,
2-Amino-4-N-methylpiperazino-6-(4-methoxyphenyl)pteridin,
2-Amino-4-methylpiperazino-6-(3,4-dimethoxyphenyl)pteridin,
2-Amino-4-cyclopentylamino-6-(4-methoxyphenyl)pteridin,
2-Amino-4-piperazino-6-phenylpteridin,
2-Amino-4-piperazino-6-(4-chlorophenyl)pteridin,
2-Amino-4-piperazino-6-4(methoxyphenyl)pteridin,
2-Amino-4-piperazino-6-(3,4-dimethoxyphenyl)pteridin,
2-Amino-4-dibenzylamino-6-(3,4,5-trimethoxyphenyl)pteridin,
2-Amino-4-morpholino-6-(3,4,5-trimethoxyphenyl)pteridin,
2-Amino-4-adamantylamino-6-(3,4,5-trimethoxyphenyl)pteridin,
2-Amino-4-adamantylamino-6-naphtylpteridin,
2-Amino-4-adamantylamino-6-(3,4,5-trimethoxyphenyl)pteridin,
2-Amino-4-adamantylamino-6-naphtylpteridin,
2-Amino-4-morpholino-6-(3,4-formyliden-3,4-dihydroxyphenyl) pteridin,
2-Amino-4-dimethylamino-6-(3,4-formyliden-3,4-dihydroxyphenyl) pteridin,
2-Amino-4-cyclopentylamino-6-(3,4-dimethoxyphenyl)pteridin,
2-Amino-4-dimethylamino-6-(3,4-dimethoxyphenyl)pteridin,
2-Amino-4-dimethylamino-6-methylpteridin,
2-Amino-4-ethoxy-6-phenylpteridin,
2-Amino-4-propylamino-6-phenylpteridin,
2-Amino-4-propylamino-6-(3,4-dimethoxyphenyl)pteridin,
2-Acetamido-4-hydroxy-6-(3,4-dimethoxyphenyl)pteridin,
2-Acetamido-4-i-propoxy-6-(3,4-dimethoxyphenyl)pteridin, und
2-Amino-4-ethoxy-6-(3,4-dimethoxyphenyl)pteridin.

**19.** Verbindung nach Anspruch 13, die ein saures Additionssalz eines Pteridin-Derivates mit der allgemeinen Formel (1) ist, wobei die Säure aus der Gruppe ausgewählt ist, die aus Halogenwasserstoffsäuren; Schwefelsäure, Salpetersäure, Phosphorsäure, organische Säuren beispielsweise Essigsäure, Propansäure, Hydroxyessigsäure, 2-Hydroxypropansäure, 2-Oxopropansäure, Ethandisäure, Propandisäure, Butandisäure, (Z)-2-Butendisäure, (E)-2-Butendisäure, 2-Hydroxybutandisäure, 2,3-Dihydroxybutandisäure, 2-Hydroxy-1,2,3-Propantricarbonäure, Methansulfonsäure, Ethansulfonsäure, Benzolsulfonsäure, 4-Methylbenzolsulfonsäure, Cyclohexansulfaminsäure, 2-Hydroxybenzoesäure und 4-Amino-2-Hydroxybenzoesäuren, besteht.

**20.** Verbindung nach Anspruch 13, die eine Basenadditionsalzform eines Pteridin-Derivates mit der allgemeinen Formel (1) ist, wobei die Basenadditionssalzform aus der Gruppe ausgewählt ist, die aus die aus Natrium-, Kalium- und Kalziumsalzen besteht; und Salzen mit pharmazeutisch verträglichen Aminen, beispielsweise Ammoniak, Alkylamin, Benzathin, N-Methyl-D-Glucamin und Hydrabamin und Aminosäuren beispielsweise Arginin und Lysin.

**21.** Verbindung nach Anspruch 13, die ein Solvat eines Pteridin-Derivates mit der allgemeinen Formel (I) ist.

**22.** Verbindung nach Anspruch 21, wobei das Solvat ein Hydrat oder ein Alkoholat ist.

**23.** Verwendung einer Verbindung nach einem der Ansprüche 13 bis 22 zur Herstellung eines Medikamentes zur Behandlung von Autoimmunkrankheiten.

**24.** Verwendung einer Verbindung nach einem der Ansprüche 13 bis 22 für die Herstellung eines Medikamentes zur Vorbeugung von Transplantatabstoßungen.

**25.** Verwendung einer Verbindung nach einem der Ansprüche 13 bis 22 für die Herstellung eines Medikamentes zur Behandlung von entzündlichen Erkrankungen.

**26.** Verfahren zum Selektieren immunsuppressiver Mittel aus Verbindungen gemäß einem der Ansprüche 13 bis 22 durch eine Kombination von zumindest drei Testsystemen auf Grundlage von MLR, aCD3 und aCD28.

**27.** Pharmazeutische Zusammensetzung, die eine Verbindung nach einem der Ansprüche 13 bis 22 und einen pharmazeutisch verträglichen Träger umfasst.

**Revendications**

**1.** Composition pharmaceutique pour emploi dans le traitement de désordres auto-immunes ou pour le traitement et / ou la prévention de rejets de transplantations et / ou pour le traitement de maladies inflammatoires comprenant comme principe actif au moins un dérivé de ptéridine ayant la formule générale :

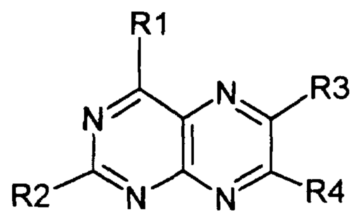

(I)

dans laquelle :

$R_1$ est hydroxylamino , (mono- ou di) $C_{1-7}$ alcoylamino , (mono- or di) $C_{1-7}$ alcoyloxyamino , (mono- or di) arylamino , (mono- or di) $C_{3-10}$ cycloalcoylamino , (mono- or di) hydroxy $C_{1-7}$ alcoylamino , (mono- or di) $C_{1-4}$ alcoyl-arylamino , mercapto $C_{1-7}$ alcoyl , $C_{1-7}$ alcoyloxy ou un composé hétérocyclique saturé ou insaturé contenant au moins un azote et le cas échéant substitué par un ou plusieurs $C_{1-4}$ alcoyle , hydroxy $C_{1-4}$ alcoyle , $C_{1-4}$ alcoyloxy , halo , hydroxyle , hydroxycarbonyle , $C_{1-4}$ alcoyloxycarbonyle ,

$R_2$ est amino , hydroxylamino , (mono- or di) $C_{1-7}$ alcoylamino , (mono- or di) $C_{1-7}$ alcoyloxyamino , (mono- or di) arylamino , (mono- or di) $C_{3-10}$ cycloalcoylamino , (mono- or di) hydroxy $C_{1-7}$ alcoylamino , (mono- or di) $C_{1-4}$ alcoyl-arylamino , mercapto $C_{1-7}$ alcoyl , $C_{1-7}$ alcoyloxy ou un composé hétérocyclique saturé ou insaturé contenant au moins un azote et le cas échéant substitué par un ou plusieurs $C_{1-4}$ alcoyle , hydroxy $C_{1-4}$ alcoyle , $C_{1-4}$ alcoyloxy , halo , hydroxyle , hydroxycarbonyle , $C_{1-4}$ alcoyloxycarbonyle , $R_3$ est un groupe aryle non substitué , monosubstitué ou disubstitué (dans lequel le substituant peut être , mais n' est pas limité à , halogène , $C_{1-4}$ alcoxy, $C_{1-4}$ alcoyle) , un groupe aryle lié à l'anneau ptéridine par un espaceur aliphatique saturé ou insaturé qui peut être halogéné ou hydroxylé , un substituant aliphatique qui peut contenir une fonction éther , une fonction alcool, des fonctions amino substituées ou non substituées ou $C_{1-4}$ alcoyloxy,

$R_4$ est hydrogène , alcoyle , alcoxy , aryle substitué ou non substitué ,

ou un sel d' addition pharmaceutiquement acceptable ou une forme isomère stéréochimique de celui-ci .

**2.** Composition pharmaceutique conforme à la revendication 1 , dans laquelle $R_1$ is di($C_{1-4}$ alkyl) amino, morpholinyl, pipéridinyl, pipérazinyl, $C_{1-4}$ alkylpipérazinyl, pyrrolidinyl or benzylamine.

**3.** Composition pharmaceutique conforme à la revendication 1 ou 2 , dans laquelle $R_2$ est ammonium , hydroxylammonium , (mono-or di) hydroxy $C_{1-7}$ alcoylammonium .

**4.** Composition pharmaceutique conforme à la revendication 1 , 2 ou 3 , dans laquelle $R_3$ est benzyle , phényle , styryle , phényl-$C_{1-4}$ alcoyloxy , phényl ($C_{1-4}$ alcoyle) le cas échéant substitué par un ou plusieurs $C_{1-4}$ alcoyle ou halo .

**5.** Composition pharmaceutique conforme à l' une quelconque des revendications 1 à 4 , dans laquelle $R_4$ est hy-

drogène ou $C_{1-4}$ alcoyle .

**6.** Composition pharmaceutique conforme à l' une quelconque des revendications 1 à 5 , dans laquelle le dérivé de ptéridine est un composé choisi parmi le groupe comprenant :

2-amino-4-pentoxy-6-styrylptéridine ,
2-amino-4-n-pentoxy-6-(1,2-dibromo-2-phényléthyl) ptéridine ,
2-amino-4-méthoxy-6-styryl-7-méthoxyptéridine ,
2-amino-4-méthoxy-6-(1,2-dihydroxypropyl) ptéridine ,
2-amino-4-hydroxylamino-6-phénylptéridine ,
2-amino-4-diméthylamino-6-phénylptéridine ,
2-amino-4-diméthylamino-6-(4-tolyl) ptéridine ,
2-amino-4-diméthylamino-6-(4-méthoxyphényl) ptéridine ,
2-amino-4-diéthylamino-6-phénylptéridine ,
2-amino-4-diéthylamino-6-(4-chlorophényl) ptéridine ,
2-amino-4-diéthylamino-6-(4-méthoxyphényl) ptéridine ,
2-amino-4-diéthylamino-6-(3,4-diméthoxyphényl) ptéridine,
2-amino-4-dibenzylamino-6-phényl ptéridine ,
2-amino-dibenzylamino-6-(4-chlorophényl) ptéridine ,
2-amino-4-dibenzylamino-6-(4-méthoxyphényl) ptéridine ,
2-amino-4-dibenzylamino-6-(3,4-diméthoxyphényl) ptéridine ,
2-amino-4-dipropylamino-6-phénylptéridine ,
2-amino-4-dipropylamino-6-(4-chlorophényl) ptéridine ,
2-amino-4-dipropylamino-6-(4-méthoxyphényl) ptéridine ,
2-amino-4-dipropylamino-6-(3,4-diméthoxyphényl) ptéridine ,
2-amino-4-morpholino-6-phénylptéridine ,
2-amino-4-morpholino-6-(4-chlorophényl) ptéridine ,
2-amino-4-morpholino-6-(4-méthoxyphényl) ptéridine ,
2-amino-4-morpholino-6-(3,4-diméthoxyphényl) ptéridine ,
2-amino-4-pipéridino-6-phénylptéridine ,
2-amino-4-pipéridino-6-(4-chlorophényl) ptéridine ,
2-amino-4-pipéridino-6-(4-méthoxyphényl) ptéridine ,
2-amino-4-pipéridino-6-(3,4-diméthoxyphényl) ptéridine ,
2-amino-4-N-méthylpipérazino-6-phénylptéridine ,
2-amino-4-N-méthylpipérazino-6-(4-chlorophényl) ptéridine ,
2-amino-4-N-méthylpipérazino-6-(4-méthoxyphényl) ptéridine ,
2-amino-4-méthylpipérazino-6-(3,4-diméthoxyphényl) ptéridine , 2-amino-4-cyclopentylamino-6-(4-méthoxyphényl) ptéridine ,
2-amino-4-pipérazino-6-phénylptéridine ,
2-amino-4-pipérazino-6-(4-chlorophényl) ptéridine ,
2-amino-4-pipérazino-6-(4-méthoxyphényl) ptéridine ,
2-amino-4-pipérazino-6-(3,4-diméthoxyphényl) ptéridine ,
2-amino-4-dibenzylamino-6-(3,4,5-triméthoxyphényl) ptéridine ,
2-amino-4-morpholino-6-(3,4,5-triméthoxyphényl) ptéridine ,
2-amino-4-adamantylamino-6-(3,4,5-triméthoxyphényl) ptéridine,
2-amino-4-adamantylamino-6-naphtylptéridine ,
2-amino-4-adamantylamino-6-(3,4,5-triméthoxyphényl) ptéridine ,
2-amino-4-adamantylamino-6-naphtylptéridine ,
2-amino-4-morpholino-6-(3,4-formylidene-3,4-dihydroxyphényl) ptéridine ,
2-amino-4-diméthylamino-6-(3,4-formylidene-3,4-dihydroxyphényl) ptéridine ,
2-amino-4-cyclopentylamino-6-(3,4-diméthoxyphényl) ptéridine ,
2-amino-4-diméthylamino-6-(3,4-diméthoxyphényl) ptéridine ,
2-amino-4-diméthylamino-6-méthylptéridine ,
2-amino-4-éthoxy-6-phénylptéridine ,
2-amino-4-propylamino-6-phénylptéridine ,
2-amino-4-propylamino-6-(3,4-diméthoxyphényl) ptéridine ,
2-acetamido-4-hydroxy-6-(3,4-diméthoxyphényl) ptéridine ,
2-acetamido-4-i-propoxy-6-(3,4-diméthoxyphényl) ptéridine , et

2-amino-4-éthoxy-6-(3,4-diméthoxyphényl) ptéridine.

**7.** Composition pharmaceutique conforme à l' une quelconque des revendications 1 à 6 , comprenant en outre un ou plusieurs autres immuno-suppresseurs .

**8.** Composition pharmaceutique conforme à la revendication 7 , dans laquelle le(s)dit(s) un ou plusieurs autres immuno-suppresseurs sont choisis parmi le groupe comprenant cyclosporine A , tacrolimus , rapamycine , leflunomide , mofetil , méthylxanthines et 8-phénylxanthines .

**9.** Composition pharmaceutique conforme à l' une quelconque des revendications 1 à 8 , comprenant comme principe actif un sel d' addition acide non toxique et thérapeutiquement actif d' un dérivé de ptéridine ayant la formule générale (I) , dans lequel ledit acide est choisi parmi le groupe constitué des acides hydrohalogénés ; l' acide sulfurique ; l' acide nitrique ; l' acide phosphorique ; des acides organiques tels que les acides acétique, propanoique , hydroxyacétique , 2-hydroxypropanoique , 2-oxopropanoique , éthanedioique , propanedioique , butanedioique , (Z)-2-butènedioique , (E)2-butènedioique , 2-hydroxybutanedioique , 2,3-dihydroxybutanedioique , 2-hydroxy-1,2,3-propanetricarboxylique , méthanesulfonique , éthanesulfonique , benzènesulfonique , 4-méthylbenzènesulfonique, cyclohexanesulfamique , 2-hydroxybenzoique et 4-amino-2-hydroxybenzoique .

**10.** Composition pharmaceutique conforme à l' une quelconque des revendications 1 à 8 , comprenant comme principe actif une forme sel d' addition basique non toxique et thérapeutiquement actif d' un dérivé de ptéridine ayant la formule générale (I) , dans lequel ladite forme sel d' addition basique est choisie parmi le groupe constitué des sels de sodium , potassium et calcium ; et les sels avec des amines pharmaceutiquement acceptables telles que l' ammoniaque , des alcoylamines , la benzathine , la N-méthyl-D-glucamine et l' hydrabamine et des acides aminés tels que l' arginine et la lysine.

**11.** Composition pharmaceutique conforme à l' une quelconque des revendications 1 à 8 , comprenant comme principe actif un solvate d' un dérivé de ptéridine derivative ayant la formule générale (I) .

**12.** Composition pharmaceutique conforme à la revendication 11 , dans laquelle ledit solvate est un hydrate ou un alcoolate .

**13.** Composé ayant la formule générale :

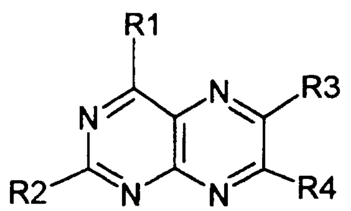

(I)

dans laquelle :

$R_1$ est hydroxylamino , (mono- ou di) $C_{1-7}$ alcoylamino , (mono- or di) $C_{1-7}$ alcoyloxyamino , (mono- or di) arylamino , (mono- or di) $C_{3-10}$ cycloalcoylamino , (mono- or di) hydroxy $C_{1-7}$ alcoylamino , (mono- or di) $C_{1-4}$ alcoyl-arylamino , mercapto $C_{1-7}$ alcoyl , $C_{1-7}$ alcoyloxy ou un composé hétérocyclique saturé ou insaturé contenant au moins un azote et le cas échéant substitué par un ou plusieurs $C_{1-4}$ alcoyl , hydroxy $C_{1-4}$ alcoyl , $C_{1-4}$ alcoyloxy , halo , hydroxyle , hydroxycarbonyle , $C_{1-4}$ alcoyloxycarbonyle ,

$R_2$ est amino , hydroxylamino , (mono- or di) $C_{1-7}$ alcoylamino , (mono- or di) $C_{1-7}$ alcoyloxyamino , (mono- or di) arylamino , (mono- or di) $C_{3-10}$ cycloalcoylamino , (mono- or di) hydroxy $C_{1-7}$ alcoylamino , (mono- or di) $C_{1-4}$ alcoyl-arylamino , mercapto $C_{1-7}$ alcoyl , $C_{1-7}$ alcoyloxy ou un composé hétérocyclique saturé ou insaturé contenant au moins un azote et le cas échéant substitué par un ou plusieurs $C_{1-4}$ alcoyle , hydroxy $C_{1-4}$ alcoyle , $C_{1-4}$ alcoyloxy , halo , hydroxyle , hydroxycarbonyle , $C_{1-4}$ alcoyloxycarbonyle ,

$R_3$ est un groupe aryle non substitué , monosubstitué ou disubstitué (dans lequel le substituant peut être , mais n' est pas limité à , halogène , $C_{1-4}$ alcoxy, $C_{1-4}$ alcoyle) , un groupe aryle lié à l' anneau ptéridine par un espaceur aliphatique saturé ou insaturé qui peut être halogéné ou hydroxylé , un substituant aliphatique qui peut contenir une fonction éther , une fonction alcool, des fonctions amino substituées ou non substituées ou $C_{1-4}$ alcoyloxy ,

$R_4$ is hydrogène , alcoyle , alcoxy , aryle substitué ou non substitué ,

ou un sel d' addition pharmaceutiquement acceptable ou une forme isomère stéréochimique de celui-ci , à l' exception de :

- 2-amino-4-méthylamino-6-phényl-7-phénylptéridine ,
- 2-amino-4-diméthylamino-6-phényl-7-phénylptéridine ,
- 2-méthylamino-4-méthylamino-6-méthyl-7-méthylptéridine ,
- 2-méthylamino-4-méthylamino-6-phénylptéridine ,
- 2-méthylamino-4-méthylamino-6-phényl-7-phénylptéridine ,
- 2-méthylamino-4-méthylamino-6-(*o*-chlorophényl)-7-(*o*-chlorophényl)ptéridine ,
- 2-méthylamino-4-méthylamino-6-(m-chlorophényl)-7-(m-chlorophényl)ptéridine ,
- 2-méthylamino-4-méthylamino-6-(*p*-chlorophényl)-7-(*p*-chlorophényl)ptéridine ,
- 2-méthylamino-4-méthylamino-6-(*p*-méthoxyphényl)-7-(*p*-méthoxyphényl)ptéridine ,
- 2-méthylamino-4-diméthylamino-6-phényl-7-phénylptéridine ,
- 2-éthylamino-4-méthylamino-6-phényl-7-phénylptéridine ,
- 2-diméthylamino-4-éthoxy-6-phénylptéridine ,
- 2-diméthylamino-4-méthylamino-6-phényl-7-phénylptéridine ,
- 2-diméthylamino-4-méthylamino-6-phényl-7-(*p*-chlorophényl)ptéridine ,
- 2-diméthylamino-4-diméthylamino-6-propyl-7-propylptéridine ,
- 2-diméthylamino-4-diméthylamino-6-phénylptéridine ,
- 2-diméthylamino-4-diméthylamino-6-phényl-7-phénylptéridine ,
- 2-diméthylamino-4-pipéridino-6-phényl-7-phénylptéridine ,
- 2-diméthylamino-4-morpholino-6-phényl-7-phénylptéridine ,
- 2-amino-4-éthoxy-6,7-diphénylptéridine ,
- 2-amino-4-diéthylamino-6,7-diphénylptéridine , et
- 2-amino-4-pipéridino-6,7-diphénylptéridine.

**14.** Composé conforme à la revendication 13 , dans lequel $R_1$ est di($C_{1-4}$ alcoyl) amino , morpholinyl , pipéridinyl , pipérazinyl , $C_{1-4}$ alcoylpipérazinyl, pyrrolidinyl ou benzylamine .

**15.** Composé conforme à la revendication 13 ou la revendication 14 , dans lequel $R_2$ est ammonium , hydroxyammonium , (mono-or di) hydroxy $C_{1-7}$ alcoylammonium .

**16.** Composé conforme à l' une quelconque des revendications 13 à 15 , dans lequel $R_3$ est benzyle , phényle , styryle , phényl-$C_{1-4}$ alcoyloxy , phényl ($C_{1-4}$ alcoyl) le cas échéant substitué par un ou plusieurs $C_{1-4}$ alcoyle ou halo .

**17.** Composé conforme à l' une quelconque des revendications 13 à 16 , dans lequel $R_4$ est hydrogène ou $C_{1-4}$ alcoyle .

**18.** Composé conforme à la revendication 13 , choisi parmi le groupe comprenant :

2-amino-4-pentoxy-6-styrylptéridine ,
2-amino-4-n-pentoxy-6-(1,2-dibromo-2-phényléthyl) ptéridine ,
2-amino-4-méthoxy-6-styryl-7-méthoxyptéridine ,
2-amino-4-diméthylamino-6-phénylptéridine ,
2-amino-4-diméthylamino-6-(4-tolyl) ptéridine ,
2-amino-4-diméthylamino-6-(4-méthoxyphényl) ptéridine ,
2-amino-4-diéthylamino-6-phénylptéridine ,
2-amino-4-diéthylamino-6-(4-chlorophényl) ptéridine ,
2-amino-4-diéthylamino-6-(4-méthoxyphényl) ptéridine ,
2-amino-4-diéthylamino-6-(3,4-diméthoxyphényl) ptéridine ,
2-amino-4-dibenzylamino-6-phényl ptéridine ,

2-amino-dibenzylamino-6-(4-chlorophényl) ptéridine ,
2-amino-4-dibenzylamino-6-(4-méthoxyphényl) ptéridine ,
2-amino-4-dibenzylamino-6-(3,4-diméthoxyphényl) ptéridine ,
2-amino-4-dipropylamino-6-phénylptéridine ,
2-amino-4-dipropylamino-6-(4-chlorophényl) ptéridine ,
2-amino-4-dipropylamino-6-(4-méthoxyphényl) ptéridine ,
2-amino-4-dipropylamino-6-(3,4-diméthoxyphényl) ptéridine ,
2-amino-4-morpholino-6-phénylptéridine ,
2-amino-4-morpholino-6-(4-chlorophényl) ptéridine ,
2-amino-4-morpholino-6-(4-méthoxyphényl) ptéridine ,
2-amino-4-morpholino-6-(3,4-diméthoxyphényl) ptéridine ,
2-amino-4-pipéridino-6-phénylptéridine ,
2-amino-4-pipéridino-6-(4-chlorophényl) ptéridine ,
2-amino-4-pipéridino-6-(4-méthoxyphényl) ptéridine ,
2-amino-4-pipéridino-6-(3,4-diméthoxyphényl) ptéridine ,
2-amino-4-N-méthylpipérazino-6-phénylptéridine ,
2-amino-4-N-méthylpipérazino-6-(4-chlorophényl) ptéridine ,
2-amino-4-N-méthylpipérazino-6-(4-méthoxyphényl) ptéridine ,
2-amino-4-méthylpipérazino-6-(3,4-diméthoxyphényl) ptéridine ,
2-amino-4-cyclopentylamino-6-(4-méthoxyphényl) ptéridine ,
2-amino-4-pipérazino-6-phénylptéridine ,
2-amino-4-pipérazino-6-(4-chlorophényl) ptéridine ,
2-amino-4-pipérazino-6-(4-méthoxyphényl) ptéridine ,
2-amino-4-pipérazino-6-(3,4-diméthoxyphényl) ptéridine ,
2-amino-4-dibenzylamino-6-(3,4,5-triméthoxyphényl) ptéridine ,
2-amino-4-morpholino-6-(3,4,5-triméthoxyphényl) ptéridine ,
2-amino-4-adamantylamino-6-(3,4,5-triméthoxyphényl) ptéridine ,
2-amino-4-adamantylamino-6-naphtylptéridine ,
2-amino-4-adamantylamino-6-(3,4,5-triméthoxyphényl) ptéridine ,
2-amino-4-adamantylamino-6-naphtylptéridine ,
2-amino-4-morpholino-6-(3,4-formylidene-3,4-dihydroxyphényl) ptéridine ,
2-amino-4-diméthylamino-6-(3,4-formylidene-3,4-dihydroxyphényl) ptéridine ,
2-amino-4-cyclopentylamino-6-(3,4-diméthoxyphényl) ptéridine ,
2-amino-4-diméthylamino-6-(3,4-diméthoxyphényl) ptéridine ,
2-amino-4-diméthylamino-6-méthylptéridine ,
2-amino-4-éthoxy-6-phénylptéridine
2-amino-4-propylamino-6-phénylptéridine ,
2-amino-4-propylamino-6-(3,4-diméthoxyphényl) ptéridine ,
2-acétamido-4-hydroxy-6-(3,4-diméthoxyphényl) ptéridine ,
2-acétamido-4-i-propoxy-6-(3,4-diméthoxyphényl) ptéridine , et
2-amino-4-éthoxy-6-(3,4-diméthoxyphényl) ptéridine .

**19.** Composé conforme à la revendication 13 , étant le sel d' addition acide d' un dérivé de ptéridine ayant la formule générale (I) , dans lequel ledit acide est choisi parmi le groupe constitué des acides hydrohalogénés ; l' acide sulfurique ; l' acide nitrique ; l' acide phosphorique ; des acides organiques tels que les acides acétique, propanoique , hydroxyacétique , 2-hydroxypropanoique , 2-oxopropanoique , éthanedioique , propanedioique , butanedioique , (Z)-2-butène-dioique , (E)2-butènedioique , 2-hydroxybutanedioique , 2,3-dihydroxybutanedioique , 2-hydroxy-1,2,3-propanetricarboxylique , méthanesulfonique , éthanesulfonique , benzènesulfonique , 4-méthylbenzènesulfonique, cyclohexanesulfamique , 2-hydroxybenzoique et 4-amino-2-hydroxybenzoique .

**20.** Composé conforme à la revendication 13 , étant la forme sel d' addition basique d' un dérivé de ptéridine ayant la formule générale (I) , dans lequel ladite forme sel d' addition basique est choisie parmi le groupe constitué des sels de sodium , potassium et calcium ; et les sels avec des amines pharmaceutiquement acceptables telles que l' ammoniaque , des alcoylamines , la benzathine , la N-méthyl-D-glucamine et l' hydrabamine et des acides aminés tels que l' arginine et la lysine .

**21.** Composé conforme à la revendication 13 , étant le solvate d' un dérivé de ptéridine ayant la formule générale (I) .

**22.** Composé conforme à la revendication 21 , dans lequel ledit solvate est un hydrate ou un alcoolate .

**23.** Utilisation d' un composé conforme à l' une quelconque des revendications 13 à 22 pour la fabrication d' un médicament pour le traitement de désordres auto-immunes.

**24.** Utilisation d' un composé conforme à l' une quelconque des revendications 13 à 22 pour la fabrication d' un médicament pour la prévention de rejets de transplantations.

**25.** Utilisation d' un composé conforme à l' une quelconque des revendications 13 à 22 pour la fabrication d' un médicament pour le traitement de maladies inflammatoires.

**26.** Méthode pour sélectionner des agents immuno-suppresseurs à partir des composés conformes à l' une quelconque des revendications 13 à 22 par une combinaison d' au moins trois systèmes de test basés sur MLR , aCD3 et aCD28.

**27.** Composition pharmaceutique comprenant un composé conforme à l' une quelconque des revendications 13 à 22 et un support pharmaceutiquement acceptable .